# EUROPEAN PATENT SPECIFICATION

(11) **EP 4 041 135 B1**
(45) Date of publication and mention of the grant of the patent: **06.11.2024**
(21) Application number: 20797615.0
(22) Date of filing: 06.10.2020
(51) Int. Cl.: A61F 2/24, A61F 2/958

(54) **BALLOONS FOR PROSTHETIC VALVE DELIVERY APPARATUS AND METHODS OF ASSEMBLY**
BALLONE FÜR EINE KLAPPENPROTHESENEINFÜHRUNGSVORRICHTUNG UND VERFAHREN ZUR ANORDNUNG
BALLONNETS POUR APPAREIL DE POSE DE VALVE PROTHÉTIQUE ET PROCÉDÉS D'ASSEMBLAGE

(30) Priority: 07.10.2019 US 201962911861 P; 24.10.2019 US 201962925722 P; 25.02.2020 US 202062981412 P; 13.07.2020 US 202063051244 P; 02.10.2020 US 202063086940 P
(43) Date of publication of application: 17.08.2022
(73) Proprietor: Edwards Lifesciences Corporation, Irvine, CA 92614-5688 (US)
(72) Inventor: LE, Tung T., Costa Mesa, CA 92626 (US); LE, Thanh Huy, Irvine, CA 92614 (US); MAK, Sovanpheap, Irvine, CA 92614 (US); SARAVIA, Maria L., Irvine, CA 92614 (US); NGUYEN, Kim D., Irvine, CA 92614 (US); TRAN, Sonny, Irvine, CA 92614 (US); LOOS, David John, Irvine, CA 92614 (US); AVERY, Neal H., Irvine, CA 92614 (US); WHITEHEAD, Haley Nicole, Irvine, CA 92614 (US); MURAD, Michael C., Irvine, CA 92614 (US); DE SILVA, Praveen, Irvine, CA 92614 (US)
(74) Representative: Eisenführ Speiser
(86) International application number: PCT/US2020/054410
(87) International publication number: WO 2021/071842

(56) References cited:
- EP-A1- 3 245 985
- EP-B1- 3 245 985
- WO-A1-2018/026904
- WO-A2-2012/048035
- US-A- 5 935 135
- US-A1- 2004 267 195
- US-A1- 2013 030 519
- US-A1- 2017 065 415
- US-B1- 6 280 412
- US-B2- 10 195 403

## Description

### CROSS-REFERENCE TO RELATED APPLICATIONS

This application claims the benefit of U.S. Provisional Application No. 62/911,861, filed October 7, 2019, U.S. Provisional Application No. 62/925,722, filed October 24, 2019, U.S. Provisional Application No. 62/981,412, filed February 25, 2020, U.S. Provisional Application No. 63/051,244, filed July 13, 2020, and U.S. Provisional Application No. 63/086,940, filed October 2, 2020.

### FIELD

The present disclosure concerns embodiments of a balloon for a transcatheter delivery device used for implanting a medical device, such as a prosthetic heart valve, and embodiments of a method for assembly a delivery device.

### BACKGROUND

Endovascular delivery devices are used in various procedures to deliver prosthetic medical devices or instruments to locations inside the body that are not readily accessible by surgery or where access without surgery is desirable. Access to a target location inside the body can be achieved by inserting and guiding the delivery device through a pathway or lumen in the body, including, but not limited to, a blood vessel, an esophagus, a trachea, any portion of the gastrointestinal tract, a lymphatic vessel, to name a few. In one specific example, a prosthetic heart valve can be mounted in a crimped state on the distal end of a delivery device and advanced through the patient's vasculature (e.g., through a femoral artery and the aorta) until the prosthetic valve reaches the implantation site in the heart. The prosthetic valve is then expanded to its functional size such as by inflating a balloon on which the prosthetic valve is mounted, or by deploying the prosthetic valve from a sheath of the delivery device so that the prosthetic valve can self-expand to its functional size.

Balloon-expandable prosthetic valves typically are preferred for replacing calcified native valves because the balloon of the delivery device can apply a sufficient expansion force to expand and anchor the frame of the prosthetic valve against the surrounding calcified tissue. In one known technique for delivering a prosthetic heart valve, the prosthetic heart valve may be crimped on a valve retaining portion of a balloon of the delivery device prior to insertion into the patient's body. The delivery device may include proximal and distal shoulders arranged inside the balloon and which retain the crimped prosthetic valve on the balloon as the delivery device is advanced through the patient's vasculature.

Forming and folding the balloon during assembly of the delivery device is a time consuming and labor-intensive process. Thus, improvements in the way the balloon is formed and assembled in the delivery device are desirable.

US 6,280,412 B1 discloses a stent-carrying balloon catheter in which a balloon is positioned within a stent for expanding the stent upon dilation of the balloon. The balloon includes a circumferential fold over a portion of the balloon itself, the fold encompassing a circumferential end portion of the stent for securing it in place until dilation of the balloon.

US 2017/0065415 A1 discloses a delivery apparatus for implanting a radially compressible and expandable prosthetic heart valve in a native heart valve of the heart comprises a handle portion, and an elongated balloon catheter shaft extending from the handle portion. The balloon catheter shaft includes a proximal end portion coupled to the handle portion and a distal end portion, and further comprises a balloon mounted on the distal end portion and configured to mount a prosthetic heart valve in a radially compressed state.

### SUMMARY

Disclosed herein are balloon catheters, balloon assemblies for balloon catheters, as well as related methods of forming balloon assemblies and balloon catheters. The balloon catheters can be used to deliver a medical device, tools, agents, or other therapy to a location within a body of a subject. In some embodiments, balloon catheters can be used to deliver an implantable medical device, such as a prosthetic valve, such as to a heart of the subject. In some embodiments, balloon catheters can be a component of a delivery apparatus that can used to deliver a prosthetic valve or other implantable medical device.

According to the invention, a balloon assembly including an inflatable balloon having an inflated state and a deflated state is proposed. The inflatable balloon in the deflated state includes a valve retaining portion, a distal tapered portion connected to a distal end of the valve retaining portion, a proximal tapered portion connected to a proximal end of the valve retaining portion, a distal leg connected to the distal end of the distal tapered portion, and a proximal leg connected to the proximal end of the proximal tapered portion. The valve retaining portion has a generally cylindrical shape and one or more axial folds. A proximal end of the distal tapered portion has a larger diameter than a distal end of the distal tapered portion and the valve retaining portion. A distal end of the proximal tapered portion has a larger diameter than a proximal end of the proximal tapered portion and the valve retaining portion. The distal tapered portion includes one or more axial folds. The proximal tapered portion includes one or more axial folds. The proximal end of the distal tapered portion includes a first radial fold connected to a second radial fold. The first and second radial folds form a pocket extending distally at the proximal end of the distal tapered portion.

In some embodiments, the balloon assembly can include a restraining member extending around an outer surface of the valve retaining portion so as to prevent the one or more axial folds of the valve retaining portion from unfolding.

In some embodiments, the balloon assembly can include a cover that encloses the inflatable balloon. The cover can include a proximal end portion, a distal end portion, and an intermediate portion between the proximal end portion and the distal end portion. The proximal end portion can define a proximal recess that is shaped to substantially match a shape of the proximal tapered portion of the inflatable balloon. The distal end portion can define a distal recess that is shaped to substantially match a shape of the distal tapered portion of the inflatable balloon. The intermediate portion can define an intermediate recess that is shaped to substantially match a shape of the valve retaining portion of the inflatable balloon.

In some embodiments, the cover can include a first cover piece and a second cover piece that are complimentarily structured such that they can be matingly assembled together for enclosing the inflatable balloon in the deflated state.

In some embodiments, the balloon assembly can include an outer sleeve extending over the cover so as to prevent the first and second cover pieces from separating from each other.

In some embodiments, the inflatable balloon can include an internal lumen extending through the tapered proximal portion, the valve retaining portion, and the tapered distal portion.

In some embodiments, the proximal tapered portion of the inflatable balloon can include a proximal end portion. The one or more axial folds of the proximal tapered portion do not extend into the proximal end portion of the proximal tapered portion.

In some embodiments, the distal tapered portion of the inflatable balloon can include a distal end portion. The one or more axial folds of the distal tapered portion do not extend into the distal end portion of the distal tapered portion.

According to the invention, the distal end of the proximal tapered portion includes a radial fold that forms an obtuse or right angle relative to the valve retaining portion.

In some embodiments, the first radial fold at the proximal end of the distal tapered portion can form an obtuse or right angle relative to the valve retaining portion, and the second radial fold can form an acute angle relative to the valve retaining portion.

Certain embodiments of the disclosure concern another balloon assembly having an inflatable balloon that has an inflated state and a deflated state. The inflatable balloon in the deflated state can include a valve retaining portion, a distal tapered portion connected to a distal end of the valve retaining portion, a proximal tapered portion connected to a proximal end of the valve retaining portion, one or more axially extending folds along at least sections of the distal tapered portion and the proximal tapered portion, a distal leg connected to the distal end of the distal tapered portion, and a proximal leg connected to the proximal end of the proximal tapered portion. The valve retaining portion can have a generally cylindrical shape and one or more axial folds. A proximal end of the distal tapered portion can have a larger diameter than a distal end of the distal tapered portion and the valve retaining portion. A distal end of the proximal tapered portion can have a larger diameter than a proximal end of the proximal tapered portion and the valve retaining portion. The inflatable balloon can be in an unassembled state apart from a delivery catheter.

In some embodiments, the balloon assembly can further include a restraining member extending around an outer surface of the valve retaining portion so as to prevent the one or more axial folds of the valve retaining portion from unfolding.

In some embodiments, the balloon assembly can further include a cover that encloses the inflatable balloon. The cover can include a proximal end portion, a distal end portion, and an intermediate portion between the proximal end portion and the distal end portion. The proximal end portion can define a proximal recess that is shaped to substantially match a shape of the proximal tapered portion of the inflatable balloon, and the distal end portion can define a distal recess that is shaped to substantially match a shape of the distal tapered portion of the inflatable balloon.

In some embodiments, the cover can include a first cover piece and a second cover piece that are complimentarily structured such that they can be matingly assembled together for enclosing the inflatable balloon.

In some embodiments, the balloon assembly can further include an outer sleeve extending over the cover so as to prevent the first and second cover pieces from separating from each other.

In some embodiments, the inflatable balloon can include an internal lumen extending through the tapered proximal portion, the valve retaining portion, and the tapered distal portion.

In some embodiments, the one or more axially extending folds do not extend into a proximal end portion of the proximal tapered portion.

In some embodiments, the one or more axially extending folds do not extend into a distal end portion of the distal tapered portion.

In some embodiments, the proximal end of the distal tapered portion can include a first radial fold connected to a second radial fold. The first and second radial folds can form a pocket extending distally at the proximal end of the distal tapered portion.

In some embodiments, the first radial fold at the proximal end of the distal tapered portion can form an obtuse or right angle relative to the valve retaining portion, and the second radial fold can form an acute angle relative to the valve retaining portion.

According to the invention, a method for assembling a delivery device is proposed. The method includes forming an inflatable balloon in a deflated state. The balloon in the deflated state has a valve retaining portion, a distal tapered portion connected to a distal end of the valve retaining portion, a proximal tapered portion connected to a proximal end of the valve retaining portion, a distal leg connected to a distal end of the distal tapered portion, and a proximal leg connected to a proximal end of the proximal tapered portion. The method further includes inserting a distal shoulder mounted on a first shaft of the delivery device through the distal leg and into the distal tapered portion of the inflatable balloon, inserting a proximal shoulder mounted on a second shaft of the delivery device through the proximal leg and into the proximal tapered portion of the inflatable balloon, securing the distal leg of the inflatable balloon to the distal shoulder, and securing the proximal leg of the inflatable balloon to the second shaft and/or the proximal shoulder.

In some embodiments, the method can further include placing a restraining member around an outer surface of the valve retaining portion.

In some embodiments, the method can further include enclosing the inflatable balloon with a cover. The cover can include a first cover piece and a second cover piece that are complimentarily structured such that they can be matingly assembled together for enclosing the inflatable balloon. The cover can define an internal recess whose geometry substantially matches a shape of the inflatable balloon in the deflated state.

In some embodiments, the method can further include placing a sleeve over the cover to prevent the first and second cover pieces from separating from each other.

In some embodiments, a proximal end of the distal shoulder can have a larger diameter than a diameter of the distal leg. Inserting the distal shoulder through the distal leg can include radially compressing the proximal end of the distal shoulder.

In some embodiments, a distal end of the proximal shoulder can have a larger diameter than a diameter of the proximal leg. Inserting the proximal shoulder through the proximal leg can include radially compressing the distal end of the proximal shoulder.

According to the invention, forming the inflatable balloon includes forming the valve retaining portion in a generally cylindrical shape, the diameter of which being smaller than both a diameter of a proximal end of the distal tapered portion and a diameter of a distal end of the proximal tapered portion.

According to the invention, forming the inflatable balloon includes forming one or more axially extending folds along at least sections of the distal tapered portion and the proximal tapered portion.

According to the invention, forming the inflatable balloon includes forming a distal end portion of the distal tapered portion, and the distal end portion can be free of any of the one or more axially extending folds.

In some embodiments, forming the inflatable balloon can include forming a proximal end portion of the proximal tapered portion, and the proximal end portion can be free of any of the one or more axially extending folds.

According to the invention, forming the inflatable balloon includes forming a radial fold at the distal end of the proximal tapered portion, and the radial fold forms an obtuse or right angle relative to the valve retaining portion.

In some embodiments, forming the inflatable balloon can include forming a radial fold at the proximal end of the distal tapered portion, and the radial fold can form an obtuse or right angle relative to the valve retaining portion.

According to the invention, forming the inflatable balloon includes forming a first radial fold connected to a second radial fold at the proximal end of the distal tapered portion. The first and second radial folds form a pocket extending distally at the proximal end of the distal tapered portion.

In some embodiments, the distal shoulder can include one or more distal shoulder fins extending at least partially radially outwardly. Inserting the distal shoulder assembly into the distal tapered portion can include snuggly placing the one or more distal shoulder fins adjacent to at least some of the one or more axially extending folds in the distal tapered portion.

In some embodiments, the proximal shoulder can include one or more proximal shoulder fins extending at least partially radially outwardly. Inserting the proximal shoulder assembly into the proximal tapered portion can include snuggly placing the one or more proximal shoulder fins adjacent to at least some of the one or more axially extending folds in the proximal tapered portion.

In some embodiments, inserting the distal shoulder into the distal tapered portion can include aligning a marker located on the first shaft with a predefined position on the valve retaining portion of the inflatable balloon or the proximal shoulder.

Certain embodiments of the disclosure concern another method for assembling a delivery device. The method can include forming an inflatable balloon in a deflated state. The balloon in the deflated state can have a distal portion, a proximal portion, and a valve retaining portion between the distal portion and the proximal portion. The method can further include forming a plurality of axially extending folds along at least sections of the distal and proximal portions of the inflatable balloon, and after forming the inflatable balloon and forming the plurality of axially extending folds, mounting the inflatable balloon in the deflated state on a delivery device.

In some embodiments, mounting the inflatable balloon on the delivery device can include inserting a distal shoulder mounted on a first shaft of the delivery device through a distal leg of the balloon and inserting a proximal shoulder mounted on a second shaft of the delivery device through a proximal leg of the balloon. The distal leg can be connected to a distal end of a tapered portion of the distal portion of the inflatable balloon, and the proximal leg can be connected to a proximal end of a tapered portion of the proximal portion of the inflatable balloon.

In some embodiments, a proximal end of the distal shoulder can have a larger diameter than a diameter of the distal leg. Inserting the distal shoulder through the distal leg can include radially compressing the proximal end of the distal shoulder.

In some embodiments, a distal end of the proximal shoulder can have a larger diameter than a diameter of the proximal leg. Inserting the proximal shoulder through the proximal leg can include radially compressing the distal end of the proximal shoulder.

In some embodiments, mounting the inflatable balloon on the delivery device can include inserting the distal shoulder into the tapered portion of the distal portion of the inflatable balloon, and inserting the proximal shoulder into the tapered portion of the proximal portion of the inflatable balloon.

In some embodiments, the distal shoulder can include one or more distal shoulder fins extending at least partially radially outwardly. Inserting the distal shoulder into the tapered portion of the distal portion can include snuggly placing the one or more distal shoulder fins adjacent to at least some of the plurality of axially extending folds in the tapered portion of the distal portion.

In some embodiments, the proximal shoulder can include one or more proximal shoulder fins extending at least partially radially outwardly. Inserting the proximal shoulder into the tapered portion of the proximal portion can include snuggly placing the one or more proximal shoulder fins adjacent to at least some of the plurality of axially extending folds in the tapered portion of the proximal portion.

In some embodiments, inserting the distal shoulder into the tapered portion of the distal portion can include aligning a marker on the first shaft with a predefined position on the valve retaining portion of the inflatable balloon or the proximal shoulder.

In some embodiments, forming the inflatable balloon can include forming a distal end portion of the distal portion, and the distal end portion can be free of any of the plurality of axially extending folds.

In some embodiments, forming the inflatable balloon can include forming a proximal end portion of the proximal portion, and the proximal end portion can be free of any of the plurality of axially extending folds.

In some embodiments, forming the inflatable balloon can include forming the valve retaining portion in a generally cylindrical shape, the diameter of which being smaller than both a diameter of a proximal end of the distal portion and a diameter of a distal end of the proximal portion.

In some embodiments, forming the inflatable balloon can include forming a radial fold at the distal end of the proximal portion, and the radial fold can form an obtuse or right angle relative to the valve retaining portion.

In some embodiments, forming the inflatable balloon can include forming a radial fold at the proximal end of the distal portion, and the radial fold can form an obtuse or right angle relative to the valve retaining portion.

In some embodiments, forming the inflatable balloon can include forming a first radial fold connected to a second radial fold at the proximal end of the distal portion. The first and second radial folds can form a pocket extending distally at the proximal end of the distal portion.

In some embodiments, mounting the inflatable balloon on the delivery device can include securing the distal leg to the distal shoulder, and securing the proximal leg to the second shaft and/or the proximal shoulder.

In some embodiments, the method can further include placing a restraining member around the valve retaining portion.

In some embodiments, the method can further include enclosing the inflatable balloon with a cover. The cover can include a first cover piece and a second cover piece that are complimentarily structured such that they can be matingly assembled together for enclosing the inflatable balloon. The cover can define an internal recess whose geometry substantially matches a shape of the inflatable balloon in the deflated state.

In some embodiments, the method can further include placing a sleeve around the cover to prevent the first and second cover pieces from separating from each other.

According to the invention, furthermore, a prosthetic valve delivery assembly including a delivery device is proposed. The delivery device can include a shaft and an inflatable balloon mounted on the shaft in a deflated state. The balloon in the deflated state can include a valve retaining portion having a generally cylindrical shape, a distal tapered portion connected to a distal end of the valve retaining portion, a proximal tapered portion connected to a proximal end of the valve retaining portion. The proximal end of the distal tapered portion can include a first radial fold connected to a second radial fold. The first and second radial folds can form a pocket extending distally at the proximal end of the distal tapered portion.

In some embodiments, the prosthetic valve delivery assembly can further include a restraining member extending around an outer surface of the valve retaining portion.

In some embodiments, the prosthetic valve delivery assembly can further include a cover. The cover can include a first cover piece and a second cover piece that are complimentarily structured such that they can be matingly assembled together for enclosing the inflatable balloon. The cover can define an internal recess whose geometry substantially matches a shape of the inflatable balloon in the deflated state.

In some embodiments, the prosthetic valve delivery assembly can further include an outer sleeve extending over the cover so as to prevent the first and second cover pieces from separating from each other.

In some embodiments, the balloon in the deflated state can include one or more axially extending folds along at least sections of the distal tapered portion and the proximal tapered portion.

In some embodiments, the distal tapered portion can include a distal end portion that is free of any of the one or more axially extending folds.

In some embodiments, the balloon can further include a distal leg connected to a distal end of the distal tapered portion and a proximal leg connected to a proximal end of the proximal tapered portion.

In some embodiments, the delivery device can include a distal shoulder mounted on the first shaft extending through the distal leg and into the distal tapered portion of the inflatable balloon, and a proximal shoulder mounted on a second shaft of the delivery device extending through the proximal leg and into the proximal tapered portion of the inflatable balloon.

In some embodiments, the distal leg can be secured to the distal shoulder and the proximal leg can be secured to the second shaft or the proximal shoulder.

In some embodiments, a proximal end of the distal shoulder can have a larger diameter than a diameter of the distal leg, and a distal end of the proximal shoulder can have a larger diameter than a diameter of the proximal leg.

In some embodiments, the distal shoulder can include one or more distal shoulder fins which extend at least partially radially outwardly and are adjacent to at least some of the one or more axially extending folds in the distal tapered portion. The proximal shoulder can include one or more proximal shoulder fins extending at least partially radially outwardly and are adjacent to at least some of the one or more axially extending folds in the proximal tapered portion.

In some embodiments, the first shaft can include a marker that aligns with a predefined position on the valve retaining portion of the inflatable balloon.

In some embodiments, the first shaft can extend through the valve retaining portion, and the first shaft can be separated from the valve retaining portion by an axially extending gap.

In some embodiments, the distal end of the proximal tapered portion can include a radial fold that forms an obtuse or right angle relative to the valve retaining portion.

In some embodiments, the first radial fold at the proximal end of the distal tapered portion can form an obtuse or right angle relative to the valve retaining portion, and the second radial fold can form an acute angle relative to the valve retaining portion.

Certain embodiments of the disclosure also concern another method for assembling a delivery device. The method can comprises forming an inflatable balloon in a deflated state, the balloon in the deflated state having a distal portion, a proximal portion, and a valve retaining portion between the distal portion and the proximal portion; placing the balloon inside of a cover; inserting a shaft of a delivery device through an opening of the cover and into the balloon while the balloon is inside the cover; and securing the balloon to a surface of the delivery device.

Certain embodiments of the disclosure concern a balloon cover for a balloon catheter. The balloon cover can include an outer shell and an inner sleeve disposed within the outer shell. The inner sleeve can have an inner surface defining a lumen that is configured to receive at least a portion of a balloon of the balloon catheter. The inner sleeve can be deformable under pressure exerted against the inner surface by the balloon upon inflation of the balloon, thereby radially expanding the lumen from a first diameter to a second diameter, wherein the second diameter is larger than the first diameter.

Certain embodiments of the disclosure also concern a medical assembly. The medical assembly can include a balloon catheter having an inflatable balloon and a balloon cover. The balloon cover can include an outer shell and an inner member disposed within the outer shell, and the inner member can have an inner surface defining a lumen. The balloon can be disposed in the lumen of the inner member. The inner member can be radially deformable when the balloon is inflated and exerts pressure radially outwardly against the inner surface of the inner member.

Certain embodiments of the disclosure further concern a method of inflating a balloon of a balloon catheter. The method can include fluidly connecting the balloon to a fluid source. The balloon can be in a deflated state and is disposed inside of a balloon cover. The method can further include injecting an inflation fluid from the fluid source into the balloon so as to at least partially inflate the balloon to a partially inflated state. The balloon cover can include an outer shell and an inner member disposed inside the outer shell and around the balloon. Inflating the balloon from the deflated state to the partially inflated state can deform a lumen of the inner member from an initial state to a deformed state. The lumen can have a first diameter in the initial state and a second diameter in the deformed state, and the second diameter can be larger than the first diameter.

Certain embodiments of the disclosure also concern a balloon cover for a balloon catheter. The balloon cover can include an outer shell and an inner sleeve disposed within the outer shell. The inner sleeve can have an inner surface defining a lumen that is configured to receive at least a portion of a balloon of the balloon catheter. The inner sleeve can include first and second separable portions, each having an interior surface defining a section of the lumen. The first portion can have longitudinal edges that are configured to matingly engage respective longitudinal edges of the second portion to form the lumen. The lumen can have a first diameter when the balloon is deflated and a second diameter when the balloon is inflated, the second diameter being larger than the first diameter.

Certain embodiments of the disclosure also concern a balloon cover for a balloon catheter. The balloon cover can include an outer shell and an inner sleeve disposed within the outer shell. The inner sleeve can have an inner surface defining a lumen that is configured to receive at least a portion of a balloon of the balloon catheter. The lumen can have a first diameter when the balloon is deflated and a second diameter when the balloon is inflated, the second diameter being larger than the first diameter. The inner sleeve can have a fixed axial length when the lumen expands from the first diameter to the second diameter.

Certain embodiments of the disclosure also concern a medical assembly. The assembly can include a balloon catheter having an inflatable balloon and a balloon cover. The balloon cover can include an outer shell and an inner member disposed within the outer shell. The inner member can have an inner surface defining a lumen. The balloon can be disposed in the lumen of the inner member. The lumen can be configured to expand to a second diameter when the balloon is inflated and contract to a first diameter when the balloon is deflated, the second diameter being larger than the first diameter.

Certain embodiments of the disclosure further concern a method of inflating a balloon of a balloon catheter. The method can include receiving the balloon disposed inside of a balloon cover, wherein the balloon is in a deflated state, and injecting an inflation fluid from a fluid source into the balloon so as to at least partially inflate the balloon to a partially inflated state. The balloon cover can include an outer shell and an inner member disposed inside the outer shell and around the balloon. Inflating the balloon from the deflated state to the partially inflated state can deform a lumen of the inner member from an initial state to a deformed state. The lumen can have a first diameter in the initial state and a second diameter in the deformed state, and the second diameter is larger than the first diameter.

Certain embodiments of the disclosure further concern a balloon cover for a balloon catheter having a first shell member and a second shell member configured to matingly engage each other so as to enclose a distal end portion of the balloon catheter. The first shell member can include a plurality of first wedges and first cavities and the second shell member can include a plurality of second wedges and second cavities. The first wedges and first cavities can be configured to interlock with the corresponding second cavities and second wedges when the first and second shells are matingly engaged with each other.

Certain embodiments of the disclosure further concern a medical assembly including a balloon catheter having an inflatable balloon, and a balloon cover including a first shell member and a second shell member. The first and second shell members can be configured to matingly engage with each other to define a lumen that is configured to receive at least the inflatable balloon. The first shell member can include a plurality of first wedges and first cavities and the second shell member can include a plurality of second wedges and second cavities. The first wedges and first cavities can be configured to interlock with the corresponding second cavities and second wedges when the first and second shells are matingly engaged with each other.

Certain embodiments of the disclosure concern a balloon cover for a balloon catheter including a first shell member and a second shell member. The first and second shell members can be configured to matingly engage with each other to define a lumen that is configured to receive a distal portion of the balloon catheter. The lumen can include a balloon section configured to receive a balloon mounted on the distal end portion of the balloon catheter. The balloon section can include a proximal compartment configured to receive a proximal portion of the balloon, a distal compartment configured to receive a distal portion of the balloon, and an intermediate compartment between the proximal and distal compartments, wherein the intermediate compartment can be configured to receive a valve retaining portion of the balloon. The distal compartment can include a proximal region, a distal region, and a middle region between the proximal and distal regions. The distal region can have a smaller diameter than the proximal region and the middle region can have a smaller diameter than the distal region.

Certain embodiments of the disclosure also concern a balloon cover for a balloon catheter including a first shell member and a second shell member, wherein the first and second shell members can be configured to matingly engage with each other so that inner walls of the first and second shell members define a lumen adapted to receive a distal portion of the balloon catheter. The lumen can include a balloon section configured to receive a balloon mounted on the distal end portion of the balloon catheter. The balloon section can include a proximal compartment configured to receive a proximal portion of the balloon, a distal compartment configured to receive a distal portion of the balloon, and an intermediate compartment between the proximal and distal compartments. The intermediate compartment can be configured to receive a valve retaining portion of the balloon. Inner walls of the first and second shell members can be shaped to create a depression in at least one portion of the balloon when the balloon is retained in the lumen.

Certain embodiments of the disclosure also concern a medical assembly including a balloon catheter having an inflatable balloon. The balloon can have a proximal portion, a distal portion, and valve retaining portion between the proximal and distal portions. The assembly can also include a compression member configured create at least one depression in the balloon.

Certain embodiments of the disclosure also concern a balloon cover assembly for a balloon catheter including a cover body defining a lumen configured to receive a balloon folded on a distal portion of the balloon catheter. The balloon can include a proximal portion, a distal portion, and a valve retaining portion connecting the proximal and distal portions. The assembly can further include at least one compression member configured to radially compress at least one portion of the balloon.

Certain embodiments of the disclosure also concern a method including folding a balloon on a distal portion of a delivery device when the balloon is in a deflated state. The balloon can have a proximal portion, a distal portion, and a valve retaining portion connecting the proximal and distal portions. A distal end of the distal portion can have a smaller diameter than a proximal end of the distal portion, a distal end of the proximal portion can have a larger diameter than a proximal end of the proximal portion, and the valve retaining portion can have a smaller diameter than the proximal end of the distal portion and the distal end of the proximal portion. The method can further include radially compressing the distal portion of the balloon at a location between the distal and proximal ends of the distal portion.

The foregoing and other objects, features, and advantages of the invention will become more apparent from the following detailed description, which proceeds with reference to the accompanying figures.

### BRIEF DESCRIPTION OF THE DRAWINGS

FIG. 1 is a perspective view of a prosthetic heart valve, according to one embodiment.
FIG. 2A is a perspective view of a prosthetic heart valve, according to another embodiment.
FIG. 2B is a perspective view of the prosthetic valve of FIG. 2A with the components on the outside of the frame shown in transparent lines for purposes of illustration.
FIG. 3 is a perspective view of a delivery for a prosthetic heart valve, according to one embodiment.
FIG. 4 is an enlarged, cross-sectional view of the distal end portion of the delivery apparatus of FIG. 3.
FIG. 5 is an enlarged, side elevation view of the distal end portion of the delivery apparatus of FIG. 3.
FIG. 6 is an enlarged, cross-sectional view of the distal end portion of a delivery apparatus, according to another embodiment.
FIG. 7 shows a section of the distal end portion of the delivery apparatus shown in FIG. 6.
FIG. 8 shows the distal end portion of the delivery apparatus of FIG. 6 and a cover assembly, shown in cross-section, enclosing the balloon of the delivery apparatus.
FIG. 9 is an exploded view of the cover assembly of FIG. 8.
FIG. 10 shows the cover assembly of FIG. 8 is a disassembled state with the distal end portion of the delivery apparatus placed in one of the cover portions of the cover assembly.
FIG. 11 is a side elevation view of a balloon for a delivery device, according to another embodiment.
FIG. 12 is a side elevation view of the balloon of FIG. 11 nested inside a balloon cover, according to one embodiment.
FIG. 13 is a perspective view showing the balloon cover in an open position with the balloon inside the cover.
FIG. 14 is an illustration of method for assembling a delivery device using the balloon of FIG. 11, according to one embodiment.
FIG. 15 is a side cross-sectional view of a delivery device formed by the method shown in FIG. 14.
FIG. 16 is a side elevation view of the delivery device of FIG. 15.
FIG. 17 is a perspective view of a balloon cover, according to another embodiment, shown in an open state.
FIG. 18 is a perspective view of a portion of a balloon catheter where a balloon mounted on the balloon catheter is disposed inside the balloon cover of FIG. 17 shown in a closed state.
FIG. 19 is a longitudinal cross-sectional view of the portion of the balloon catheter including the balloon and the balloon cover depicted in FIG. 18.
FIG. 20 is a perspective view of a balloon cover, according to an alternative embodiment, shown in an open state.
FIG. 21 is a longitudinal cross-sectional view of the balloon cover depicted in FIG. 20, shown in a closed state.
FIG. 22A is a perspective view of a distal end portion of a balloon catheter received in a balloon cover, according to another embodiment, with the balloon cover shown in section.
FIG. 22B is a cross-sectional view of the distal end portion of the balloon catheter received in the balloon cover depicted in FIG. 22A.
FIG. 23A is a perspective view of the balloon cover of FIG. 22A, showing two shell members of the balloon cover separated from each other to define an open state of the balloon cover.
FIG. 23B is a cross-sectional view of the balloon cover depicted in FIG. 23A, showing the shell members in a closed state.
FIG. 24 is a side elevation view of a distal end portion of a delivery device comprising a folded balloon, according to another embodiment.
FIG. 25 is a side elevation view of the delivery device of FIG. 25 showing the folded balloon having a different shape.
FIG. 26 is a perspective view of a balloon cover configured to retain the balloon depicted in FIG. 25, according to one embodiment, wherein two shell members of the balloon cover are separated from each other so that the balloon cover is in an open state.
FIG. 27 is a cross-sectional view of the balloon cover depicted in FIG. 26, wherein the two shell members are in a closed state.
FIG. 28 is a cross-sectional view of a distal portion of the balloon cover in the closed state, wherein a distal end portion of a delivery device and a balloon are retained within the balloon cover.
FIG. 29 is a side elevation view of a balloon folded on a distal end portion of a delivery device, according to an alternative embodiment.

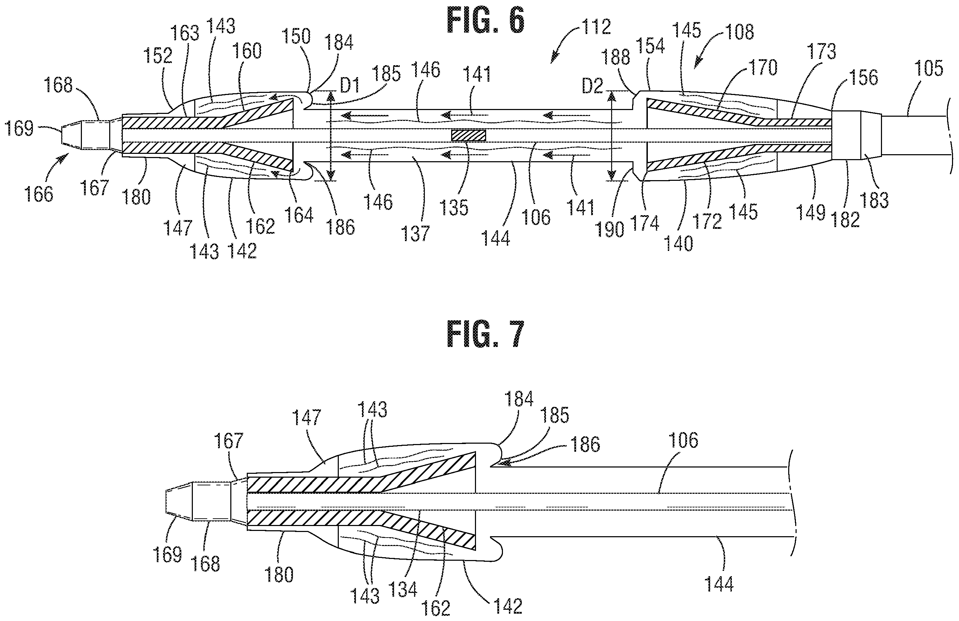

The invention relates to a balloon assembly as illustrated on fig. 6-8.

### DETAILED DESCRIPTION

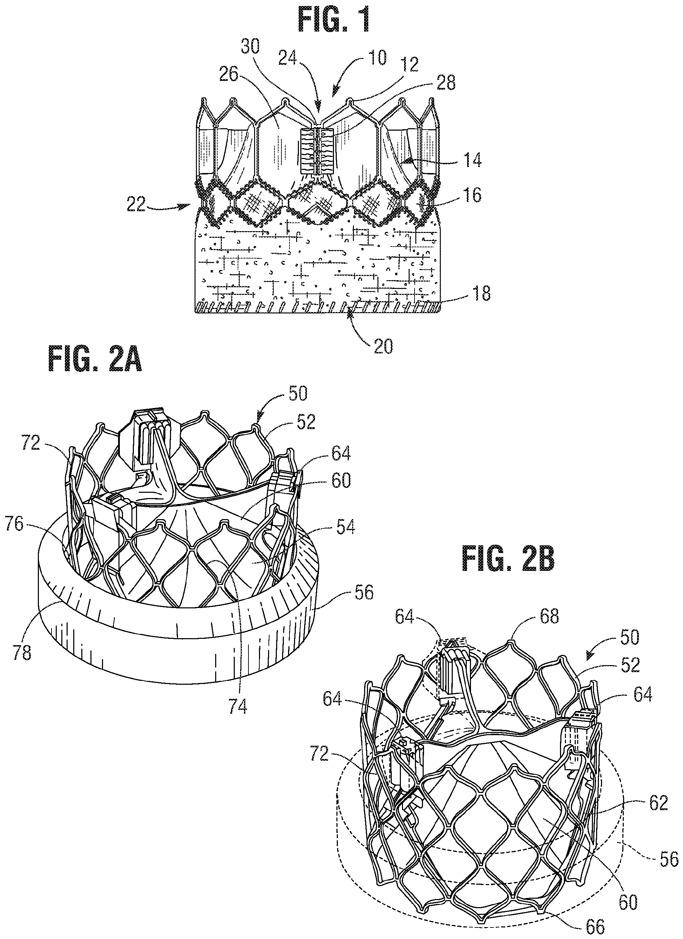

FIG. 1 shows a prosthetic heart valve 10, according to one embodiment. The illustrated prosthetic valve is adapted to be implanted in the native aortic annulus, although in other embodiments it can be adapted to be implanted in the other native annuluses of the heart (e.g., the pulmonary, mitral, and tricuspid valves). The prosthetic valve can also be adapted to be implanted in previously implanted prosthetic valves and other tubular organs or passageways in the body. The prosthetic valve 10 can have four main components: a stent or frame 12, a valvular structure 14, an inner skirt 16, and a perivalvular outer sealing member or outer skirt 18. The prosthetic valve 10 can have an inflow end portion 20, an intermediate portion 22, and an outflow end portion 24.

The valvular structure 14 can comprise three leaflets 26, collectively forming a leaflet structure, which can be arranged to collapse in a tricuspid arrangement, although in other embodiments there can be greater or fewer number of leaflets (e.g., one or more leaflets 26). The leaflets 26 can be secured to one another at their adjacent sides to form commissures 28 of the leaflet structure 14. The lower edge of valvular structure 14 can have an undulating, curved scalloped shape and can be secured to the inner skirt 16 by sutures (not shown). In some embodiments, the leaflets 26 can be formed of pericardial tissue (e.g., bovine pericardial tissue), biocompatible synthetic materials, or various other suitable natural or synthetic materials as known in the art and described in U.S. Patent No. 6,730,118.

The frame 12 can be formed with a plurality of circumferentially spaced slots, or commissure windows 30 that are adapted to mount the commissures 28 of the valvular structure 14 to the frame. The frame 12 can be made of any of various suitable plastically-expandable materials (e.g., stainless steel, a cobalt chromium alloy, etc.) or self-expanding materials (e.g., a nickel titanium alloy (NiTi), such as nitinol) as known in the art. When constructed of a plastically-expandable material, the frame 12 (and thus the prosthetic valve 10) can be crimped to a radially collapsed configuration on a delivery catheter and then expanded inside a patient by an inflatable balloon or equivalent expansion mechanism. When constructed of a self-expandable material, the frame 12 (and thus the prosthetic valve 10) can be crimped to a radially collapsed configuration and restrained in the collapsed configuration by insertion into a sheath or equivalent mechanism of a delivery catheter. Once inside the body, the prosthetic valve can be advanced from the delivery sheath, which allows the prosthetic valve to expand to its functional size.

Suitable plastically-expandable materials that can be used to form the frame 12 include, without limitation, stainless steel, a biocompatible, high-strength alloys (e.g., a cobalt-chromium or a nickel-cobalt-chromium alloys), polymers, or combinations thereof. In particular embodiments, frame 12 is made of a nickel-cobalt-chromium-molybdenum alloy, such as MP35N^{®} alloy (SPS Technologies, Jenkintown, Pennsylvania), which is equivalent to UNS R30035 alloy (covered by ASTM F562-02). MP35N^{®} alloy/UNS R30035 alloy comprises 35% nickel, 35% cobalt, 20% chromium, and 10% molybdenum, by weight. Additional details regarding the prosthetic valve 10 and its various components are described in WIPO Patent Application Publication No. WO 2018/222799.

FIG. 2A is a perspective view of a prosthetic heart valve 50, according to another embodiment. The valve 50 can have three main components: a stent or frame, 52, a valvular structure 54, and a sealing member 56. FIG. 2B is a perspective view of the prosthetic valve 50 with the components on the outside of the frame 52 (including the sealing member 56) shown in transparent lines for purposes of illustration.

Like the valvular structure 14, the valvular structure 54 can comprise three leaflets 60, collectively forming a leaflet structure, which can be arranged to collapse in a tricuspid arrangement. Each leaflet 60 can be coupled to the frame 52 along its inflow edge 62 (the lower edge in the figures; also referred to as "cusp edges" of the leaflets) and at commissures 64 of the valvular structure 54 where adjacent portions of two leaflets are connected to each other. A reinforcing element (not shown), such as a fabric strip, can be connected directly to the cusp edges of the leaflets and to the struts of the frame to couple the cusp edges of the leaflets to the frame.

Similar to the frame 12, the frame 52 can be made of any of various suitable plastically-expandable materials or self-expanding materials as known in the art and described above. The frame 52 in the illustrated embodiment comprises a plurality of circumferentially extending rows of angled struts 72 defining rows of cells, or openings, 74 of the frame. The frame 52 can have a cylindrical or substantially cylindrical shape having a constant diameter from an inflow end 66 to an outflow end 68 of the frame as shown, or the frame can vary in diameter along the height of the frame, as disclosed in U.S. Patent Publication No. 2012/0239142.

The sealing member 56 in the illustrated embodiment is mounted on the outside of the frame 52 and functions to create a seal against the surrounding tissue (e.g., the native leaflets and/or native annulus) to prevent or at least minimize paravalvular leakage. The sealing member 56 can comprise an inner layer 76 (which can be in contact with the outer surface of the frame 52) and an outer layer 78. The sealing member 56 can be connected to the frame 52 using suitable techniques or mechanisms. For example, the sealing member 56 can be sutured to the frame 52 via sutures that can extend around the struts 72 and through the inner layer 76. In alternative embodiments, the inner layer 76 can be mounted on the inner surface of the frame 52, while the outer layer 78 is on the outside of the frame 52.

The outer layer 78 can be configured or shaped to extend radially outward from the inner layer 76 and the frame 52 when the prosthetic valve 50 is deployed. When the prosthetic valve is fully expanded outside of a patient's body, the outer layer 78 can expand away from the inner layer 76 to create a space between the two layers. Thus, when implanted inside the body, this allows the outer layer 78 to expand into contact with the surrounding tissue.

Additional details regarding the prosthetic valve 50 and its various components are described in U.S. Patent Publication No. 2018/0028310.

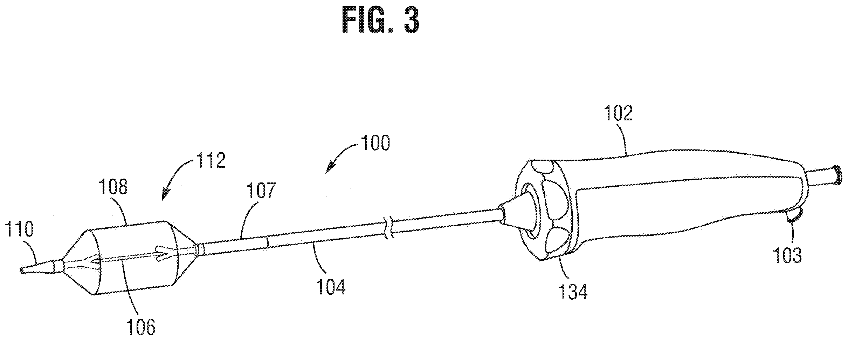

FIGS. 3-5 show various embodiments and components of a delivery device 100 (also referred to as a "delivery apparatus"), according to one embodiment, for implanting a prosthetic heart valve (e.g., a valve 10 or 50) in a patient. The delivery device 100 also can used to implant other types of expandable prosthetic medical devices (such as a stent or graft) in a patient's body.

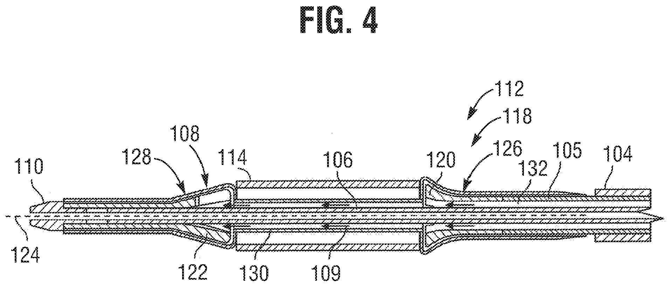

Referring to FIG. 3, the delivery device 100 in the illustrated embodiment is a balloon catheter comprising a handle 102, a steerable, outer first shaft 104 extending from the handle 102, an intermediate second shaft 105 (see FIG. 4) extending from the handle 102 coaxially through the steerable outer shaft 104, and an inner third shaft 106 extending from the handle 102 coaxially through the intermediate shaft 105 and the steerable shaft 104, an inflatable balloon 108 extending from a distal end of the intermediate shaft 105, and a nosecone 110 arranged at a distal end of the delivery device 100. A distal end portion 112 of the delivery device 100 includes the balloon 108, the nosecone 110, and a balloon shoulder assembly. A prosthetic medical device, such as a prosthetic heart valve may be mounted on a valve retaining portion of the balloon 108, as described further below with reference to FIG. 4. As described further below, the balloon shoulder assembly is configured to maintain the prosthetic heart valve or other medical device at a fixed position on the balloon 108 during delivery through the patient's vasculature.

The handle 102 can include a steering mechanism configured to adjust the curvature of the distal end portion 107 of the outer shaft 104. In the illustrated embodiment, for example, the handle 102 includes an adjustment member, such as the illustrated rotatable knob 134, which in turn is operatively coupled to the proximal end portion of a pull wire (not shown). The pull wire extends distally from the handle 102 through the outer shaft 104 and has a distal end portion affixed to the outer shaft at or near the distal end of the outer shaft 104. Rotating the knob 134 is effective to increase or decrease the tension in the pull wire, thereby adjusting the curvature of the distal end portion 107 of the outer shaft 104 and therefore the distal end portion 112 of the delivery device.

FIG. 4 shows an embodiment of the distal end portion 112 of the delivery device 100. As shown in FIG. 4, the delivery device 100 is configured to mount a prosthetic valve (e.g., prosthetic heart valve) 114 in a crimped state over the balloon 108 for insertion of the delivery device 100 and prosthetic valve 114 into a patient's vasculature.

As shown in FIG. 4, at a proximal end of the distal end portion 112, the inner shaft 106 extends distally beyond the steerable shaft 104 and the intermediate shaft 105 and through the balloon 108. The balloon 108 can be supported on a balloon shoulder assembly 118. The balloon shoulder assembly 118 includes a proximal shoulder 120 connected to a distal end of the intermediate shaft 105 and a distal shoulder 122 mounted on the inner shaft 106. The balloon 108 includes a proximal end portion 126 surrounding and/or folded over the proximal shoulder 120 and a distal end portion 128 surrounding and/or folded over the distal shoulder 122. In some embodiments, the proximal end portion 126 of the balloon 108 may be secured to the outer surface of the intermediate shaft 105. In some embodiments, the distal end portion 128 of the balloon 108 may be secured to the outer surface of the nosecone 110 (as shown), which can be mounted on or coupled to the inner shaft 106.

In the illustrated embodiment, the nosecone 110 and the distal shoulder 122 can be a one-piece or unitary component, that is, the nosecone 110 is a distal portion of the unitary component and the distal shoulder 122 is a proximal portion of the unitary component. In other embodiments, the nosecone 110 and the distal shoulder 122 can be separate components, and each can be mounted on the inner shaft 106 next to each other or at axially spaced locations.

The proximal shoulder 120 and the distal shoulder 122 are spaced apart from one another, in an axial direction relative to a central longitudinal axis 124 of the delivery device 100. As a result, the balloon 108 defines a valve-retaining portion 130 in the space that separates the proximal shoulder 120 and the distal shoulder 122 (e.g., between flared ends of the proximal shoulder 120 and the distal shoulder 122). As shown in FIG. 4, the prosthetic valve 114 can be crimped onto the valve retaining portion 130 of the balloon 108, between the proximal shoulder 120 and the distal shoulder 122, thereby preventing or reducing axial movement of the prosthetic valve 114 relative to the balloon 108 during insertion of the delivery device 100 into the patient and delivery of the prosthetic valve 114 to the target implantation site.

The outer diameter of the inner shaft 106 can be sized such that an annular space 132 is defined between the inner shaft 106 and the intermediate shaft 105 along the entire length of the intermediate shaft 105. The annular space 132 may be fluidly coupled to one or more fluid passageways of the delivery device 100 which can be fluidly connectable to a fluid source (e.g., a syringe) that can inject an inflation fluid (e.g., saline) into the delivery device. In this way, fluid from the fluid source can flow through the one or more fluid passageways, through the annular space 132, and into the balloon 108 to inflate the balloon 108 and expand and deploy the prosthetic valve 114. For example, the handle 102 can have a fluid port 103 (see FIG. 3) configured to be coupled to the fluid source. In use, inflation fluid from the fluid source can be injected into the fluid port 103, through one or more fluid passageways in the handle 102, through the annular space 132, and into the balloon 108.

FIG. 4 illustrates the flow of fluid (indicated by arrows 109) through the annular space 132 and through passages in the proximal shoulder 120 and distal shoulder 122. The fluid can then flow into the proximal and distal end portions 126, 128 of the balloon 108 to expand the valve 114.

The shafts 104, 105, and 106 of the delivery device 100 can be formed from any of various suitable materials, such as nylon, braided stainless steel wires, or a polyether block amide (commercially available as Pebax^{®}), to name a few. The shafts 104, 105, and 106 can have longitudinal sections formed from different materials in order to vary the flexibility of the shafts along their lengths. The inner shaft 106 can have an inner liner or layer formed of Teflon^{®} to minimize sliding friction with a guide wire. The shafts 104, 105, and 106 can also be axially and/or rotatably movable relative to each other and/or the handle portion 102.

Further details of the balloon shoulder assembly, the steering mechanism, and other components of the delivery device are disclosed in U.S. Publication Nos. 2007/0005131, 2009/0281619, 2013/0030519, and 2017/0065415.

In some embodiments, the delivery device 100 need not include a steering mechanism or a shaft 104 (in which case the shaft 105 is the outer shaft of the delivery device). In some embodiments, the delivery device 100 need not include a shaft 104 and can include a steering mechanism configured to adjust the curvature of the shaft 105 (e.g., the pull wire can extend through the shaft 105).

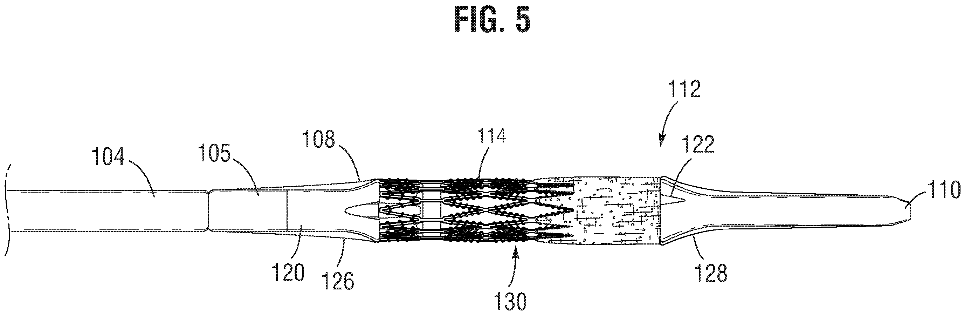

FIG. 5 shows a side view of an exterior of the distal end portion 112 of the delivery device 100, including the prosthetic valve 114 crimped on the balloon 108 mounted on the distal end portion 112 of the delivery device. As explained above, the balloon shoulder assembly including the proximal shoulder 120 and distal shoulder 122 supports the balloon 108 thereon.

As shown in FIG. 5, the balloon 108 includes the proximal end portion 126 surrounding and/or folded over the proximal shoulder 120, the distal end portion 128 surrounding and/or folded over the distal shoulder 122, and the valve retaining portion 130 located between the proximal end portion 126 and the distal end portion 128. As shown in FIG. 5, the prosthetic valve 114 is crimped on and around the valve retaining portion 130 of the balloon 108.

FIGS. 6-7 illustrates a distal end portion 112 of the delivery apparatus, according to another embodiment. In this embodiment, as shown, the balloon 108 includes a proximal portion 140, a distal portion 142, and a valve retaining portion 144 located between the proximal portion 140 and the distal portion 142.

The valve retaining portion 144 in the illustrated configuration has a generally cylindrical shape. The distal portion 142 is connected to a distal end of the valve retaining portion 144. The distal portion 142 has a tapered shape, and therefore is referred to as a "distal tapered portion." A proximal end 150 of the distal portion 142 has a larger diameter than a distal end 152 of the distal portion 142. The proximal portion 140 is connected to a proximal end of the valve retaining portion 144. The proximal portion 140 also has a tapered shape, and therefore is referred to as a "proximal tapered portion." A distal end 154 of the proximal portion 140 has a larger diameter than a proximal end 156 of the proximal portion 140.

The valve retaining portion 144 has one or more axially extending folds or pleats 146. Such axial folds 146 can be tightly compressed in order to minimize the profile of the balloon and the prosthetic heart valve 114 crimped thereon. The axial length of the valve retaining portion 144 is approximately the same as or slightly longer than the axial length of the prosthetic heart valve 114 in its crimped configuration.

As shown in FIG. 6, both the diameter of the proximal end 150 of the distal portion 142 (D1) and the diameter of the distal end 154 of the proximal portion 140 (D2) are larger than the diameter of the valve retaining portion 144. In some embodiments, the diameters D1 and D2 are configured to be about the same or slightly larger than the outer diameter of the prosthetic heart valve 114 when it is crimped over the valve retaining portion 144. Thus, when the balloon 108 and the prosthetic heart valve 114 crimped thereon are enclosed by a balloon cover (described below), the cover will not scratch or damage the frame of the prosthetic heart valve 114.

The distal tapered portion 142 can shield the valve struts against contacting the inner surface of a loader device and the inner surface of an introducer sheath during an implantation procedure device, thereby preventing or minimizing damage to those components (a loader device and an introducer sheath can be used to introduce the delivery device and the prosthetic valve into a patient's vasculature, as known in the art). In addition, the distal tapered portion 142 can reduce the insertion forces when advancing the delivery device 100 across the patient's native valve. The proximal tapered portion 140 can act as a landing support or stop for the prosthetic heart valve when being pushed through the loader, the introducer sheath and the patient's vasculature. In addition, the proximal tapered portion 140 can facilitate retrieving the delivery device from the patient's vasculature after the valve implantation.

In some embodiments, the inner shaft 106 also define a lumen that is sized to receive a guide wire that can extend coaxially through the inner shaft 106 and through the nosecone of the delivery apparatus. This allows the delivery apparatus 100 to be advanced over a guide wire previously inserted into the patient's vasculature, as known in the art.

In some embodiments, the inner shaft 106 can include a radiopaque marker 135 which is aligned with a predefined portion of the balloon 108. For example, the marker 135 can be aligned with the center of the valve retaining portion 144 of the balloon 108. The marker 135 can be used for aligning the prosthetic valve 114 with the native valve under fluoroscopy during an implantation procedure.

In the illustrated embodiment, a balloon shoulder assembly comprises a distal shoulder 160 mounted on the distal end portion of the inner shaft 106 and a proximal shoulder 170 mounted on or connected to a distal end portion of the intermediate shaft 105 (such as shown in FIG. 4), which assist in retaining the prosthetic valve 114 on the valve retaining portion 144 of the balloon during delivery through a patient's vasculature. The distal shoulder 160 can include a flared distal collar member 162 and a cylindrical shaft portion 163. The distal collar member 162 can have one or more distal shoulder fins 164, which extend at least partially radially outwardly from the shaft portion 163. The proximal shoulder 170 can include a flared proximal collar member 172 and a cylindrical shaft portion 173. The proximal collar member 172 can have one or more proximal shoulder fins 174, which extend at least partially radially outwardly from the shaft portion 173.

The shaft portion 163 of the distal collar 160 can be affixed to the distal end portion of the inner shaft 106 using any known means, such as by welding, an adhesive, mechanical fasteners, etc. The shaft portion 173 of the proximal shoulder 170 can be affixed to the distal end portion of the shaft 105 using any known means, such as by welding, an adhesive, mechanical fasteners, etc.

The outer diameter of the inner shaft 106 can be sized such that an annular space 132 (FIG. 4) is defined between the inner shaft and the intermediate shaft so as to define a fluid passageway through which an inflation fluid can flow from the handle into the balloon as previously described. As shown, the inner shaft 106 is separated from the valve retaining portion 144 of the balloon 108 by an axially extending gap 137, which allows an inflation fluid to flow from the proximal tapered portion 140 to the distal tapered portion 142 of the balloon 108.

FIG. 6 illustrates the flow of fluid (indicated by arrows 141) through the annular space 132, the proximal tapered portion 140, the axially extending gap 137, and the distal tapered portion 142 to expand the balloon 108. In addition, the balloon 108 can be deflated by withdrawing the inflation fluid from the balloon 108 through the fluid passageway described above back into a reservoir of the source of the inflation fluid.

FIG. 6 shows that the balloon 128 further includes a distal leg 180 connected to the distal end 152 of the distal tapered portion 142, and a proximal leg 182 connected to the proximal end 156 of the proximal tapered portion 140. The balloon 128 can define an internal lumen extending from the proximal leg 182, through the tapered proximal portion 140, the valve retaining portion 144, the tapered distal portion 142, and to the distal leg 180.

The proximal leg 182 can be secured to the intermediate shaft 105. For example, a proximal end portion 183 of the proximal leg 182 can be secured to an outer surface of the shaft 105. In some embodiments, the proximal end portion 183 of the proximal leg 182 can have a tapered shape that has a gradually reduced diameter in the proximal direction so as to provide a tapered transition from the proximal leg 182 to the outer surface of the shaft 105. Securing the proximal leg 182 to the shaft 105 can be achieved using any known means, including, but not limited to thermal bonding, gluing, mechanical locking, etc.

The distal leg 180 can be secured to the distal shoulder 160, such as to a distal end portion of the shaft portion 163. Securing the distal leg 180 to the distal shoulder 160 can be achieved using any known means, including, but not limited to thermal bonding, gluing, mechanical locking, etc. In FIG. 6, a small gap is shown between the distal leg 180 and the shaft portion 163 for purposes of illustration. However, it should be understood that the distal leg 180 can be in contact with and secured to the shaft portion 163 along the entire length of the distal leg 180 such that there is no gap between these two components.

In particular embodiments, as shown, the distal shoulder 160 can have a nosecone portion 166 connected to and extending distally from the shaft portion 163. The nosecone portion 166 can be integrally connected to the shaft portion 163. The nosecone portion 166 can include a first tapered portion 167, a cylindrical portion 168 extending distally from the first tapered portion 167, and a second tapered portion 169 extending distally from the cylindrical portion 168 and defining a terminal distal end of the nosecone portion and the delivery device. The first tapered portion 167 has an outer diameter that gradually reduces from the shaft portion 163 to the cylindrical portion 168. The second tapered portion has an outer diameter that gradually reduces in diameter from the cylindrical portion 168 to its terminal distal end.

The tapered portions 167, 169 can facilitate crossing of the native valve leaflets by further reducing frictional forces when advancing the delivery device through the patient's native valve.

In some embodiments, one or more sections of the nosecone portion 166, such as the cylindrical portion 168 and/or the second tapered portion 169, can be color-coded to indicate the size of the balloon 108 and the prosthetic heart valve 114 intended to be used with the delivery device 100.

The distal tapered portion 142 of the balloon includes one or more axial pleats or folds 143 when the balloon is in a deflated state ready for insertion into a patient's vasculature. The proximal tapered portion 140 of the balloon includes one or more axial pleats or folds 145 when the balloon is in a deflated state ready for insertion into a patient's vasculature. In some embodiments, the axial folds 143 or 145 can form an oblique angle relative to a longitudinal axis of the valve retaining portion 144. The folds 143, 145 reduce the overall profile of the distal end portion 112 of the delivery device to facilitate passage of the delivery device through the introducer sheath and the patient's vasculature.

In some embodiments, the distal tapered portion 142 can have a distal end section 147 that is free of any folds. For example, the one or more axial folds 143 of the distal tapered portion 142 desirably do not extend into the distal end section 147. During insertion of the delivery device through a native heart valve (e.g., a native aortic valve, which typically is calcified), the pleat-free distal end portion 147 (which has a relatively smooth outer surface) pushes open and passes through the patient's native valve first before the pleated balloon section (e.g., the section of the distal tapered portion containing the axial folds 143) passes through the native valve. Thus, the distal end section 147 creates a pleat gap that can facilitate smooth crossing of the patient's native valve when the delivery device is advanced through the native valve.

Likewise, the proximal tapered portion 140 can have a proximal end section 149 that is free of any folds. For example, the one or more axial folds 145 of the proximal tapered portion 140 desirably do not extend into the proximal end section 149. When retracting the delivery device from the native valve during an implantation procedure, such as for repositioning the prosthetic valve, the pleat-free proximal end section 149 (which has a relatively smooth outer surface) pushes open and passes through the patient's native valve first before the pleated balloon portion (e.g., the section of the proximal tapered portion containing the axial folds 145) passes through the native valve. Thus, the proximal end section 149 creates a pleat gap that can facilitate smooth crossing of the patient's native valve during retraction of the delivery device. The proximal end section 149 can also facilitate retrieval of the delivery device back through the introducer sheath after valve implantation.

In some embodiments, the axial folds 145 can extend the entire length of the proximal tapered portion 140 (i.e., no pleat gap in the proximal tapered portion). In some embodiments, the axial folds 143 can extend the entire length of the distal tapered portion 142 (i.e., no pleat gap in the distal tapered portion).

As shown in FIG. 6, the proximal tapered portion 140 of the balloon 108 can be folded around the distal end of the proximal collar member 172 so as to form an obtuse radial fold 188 that extends radially inwardly to a radial section 190 that extends radially inwardly to the proximal end of the valve retaining portion 144. The obtuse radial fold 188 provides secure nesting of the prosthetic valve 114 while facilitating passage through the introducer sheath. In the illustrated embodiment, there is a slight gap between the fold 188 and the distal end of the shoulder 170. However, in alternative embodiments, the balloon can be folded snugly around the distal end of the shoulder 170 such that there is little or no gap between the fold 188 and the shoulder 170. Moreover, the axially extending folds 145 desirably contact and extend along the proximal collar member 172 in the deflated state of the balloon.

The distal tapered portion 142 of the balloon 128 can be folded around the distal collar member 162. As shown in FIGS. 6 and 7, the distal tapered portion 142 of the balloon 108 includes a first radial fold 184 connected to a second radial fold 185 that extends to the distal end of the valve retaining portion 144. The first radial fold 184 can form an obtuse or right angle relative to the valve retaining portion 144, and the second radial fold 185 can form an acute angle relative to the valve retaining portion 144. As such, the first and second radial folds 184, 185 together form an S-shaped curve around the proximal end of the distal collar member 162. The intersection of the fold 185 and the valve retaining portion 144 can form a pocket 186 (or "tuck"). The axially extending folds 143 desirably contact and extend along the distal collar member 162 in the deflated state of the balloon.

The pocket 186 formed by radial folds 184, 185 can help keep the prosthetic heart valve 114 stable during valve deployment. As noted above, the axial length of the valve retaining portion 144 is approximately the same as or slightly longer than the axial length of the prosthetic heart valve 114 crimped thereon. As the balloon 108 is inflated during valve deployment, the prosthetic heart valve 114 is radially expanded and axially foreshortened. Without the pocket 186, such foreshortening of the prosthetic heart valve 114 during radial expansion can create an axial gap between the distal end of the prosthetic valve and the adjacent portion of the balloon, potentially causing the prosthetic heart valve 114 to shift in position. However, the radial folds 184, 185 forming the pocket 186 can mitigate such undesired shift in position of the prosthetic valve. As the inflation fluid flows through the axially extending gap 137 and into the distal tapered portion 142, the radial folds 184, 185 are unfolded under the pressure of the inflation fluid, causing the pocket 186 to unfold in the proximal direction and maintain contact against the prosthetic valve 114. Thus, the unfolding pocket provides an extra volume within the distal tapered portion 142 which can occupy or offset the axial gap that otherwise would be created by valve foreshortening. Accordingly, the radially expanding prosthetic heart valve 110, despite its axial shortening, can remain stable along the valve retaining portion 144.

In the illustrated embodiment, there is a slight gap between the folds 184, 185 and the proximal end of the distal collar member 162. However, in alternative embodiments, the balloon can be folded snugly around the proximal end of the distal collar member 162 such there is no such gap. In such embodiments, the S-shaped curve can extend around the distal collar member such that the pocket 186 is located within the interior of the distal collar member 162.

According to a configuration of the disclosure, the proximal portion 140 of the balloon 108 can be folded in the same manner as the distal portion 142 such that it also forms an S-shaped curve. According to a configuration of the disclosure, the distal portion 142 can have one obtuse fold 188 and the proximal portion 140 can have folds 184, 185 forming an S-shaped curve. According to a configuration of the disclosure, each of the proximal and distal portions 140, 142 can have one obtuse fold 188.

As noted above, the proximal collar member is placed snuggly adjacent to at least some of the axially extending folds 145 formed along the proximal tapered portion 140, and the distal collar member is placed snuggly adjacent to at least some of the axially extending folds 143 formed along the distal tapered portion 142. Such snug placement of the proximal and distal collar members next to respective balloon folds 143, 145 can help improve stability of the crimped prosthetic valve 114.

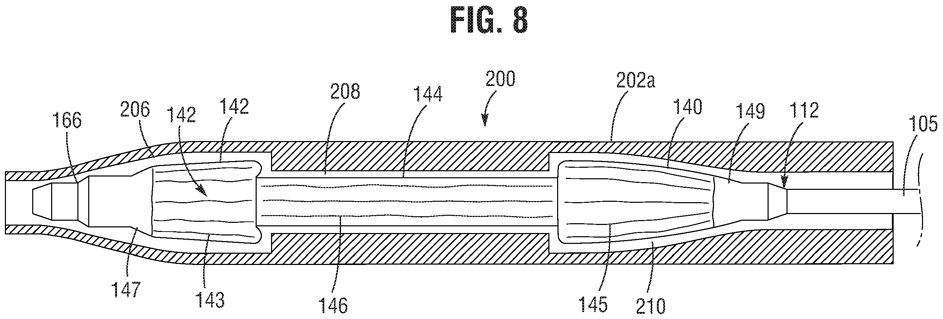

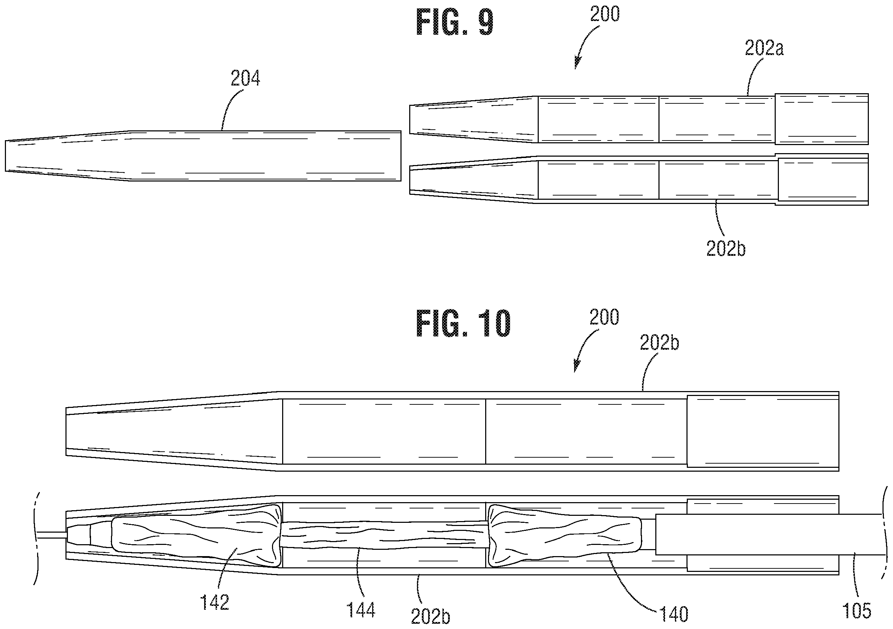

FIGS. 8-10 show a protective cover assembly 200 for protecting and maintaining the shape of the folded balloon 108 during shipping and storage prior to use, according to one embodiment. After the delivery device 100 is fully assembled, the balloon 108 can be folded at the manufacturing site so as to include the various folds described above. The cover assembly 200 can help retain the folds in the balloon until the delivery device is removed from its packaging for crimping the prosthetic valve on the balloon at the point of use.

As shown, the cover assembly 200 in the illustrated embodiment can include first and second cover portions 202a, 202b and a sleeve 204. The cover portions 202a, 202b are configured to receive and enclose the distal end portion 112 of the delivery device. The first cover portion 202a can be placed around one half of the distal end portion 112 and the second cover portion 202b can be placed around the other half of the distal end portion 112 and mate with the first cover portion 202a. The mating cover portions 202a, 202b form an enclosure that encloses or houses the distal end portion 112 of the delivery device, including the folded balloon 108, and optionally the distally end portion of the shaft 105. The sleeve 204 can be slid over the cover portions 202a, 202b to hold those components together during shipping and storage.

Each of the cover portions 202a, 202b can include a distal recess portion 206, an intermediate recess portion 208, and a proximal recess portion 210. The distal recess portion 206 can be shaped to correspond to the shape of the distal end portion 142 of the balloon and the nosecone portion 166. The intermediate recess portion 208 can be shaped to correspond to the shape of the valve retaining portion 144 of the balloon. The proximal recess portion 210 can be shaped to correspond to the shape of the proximal end portion 140 of the balloon, and optionally the distal end portion of the shaft 105.

Thus, when situated inside the cover assembly 200, the folded balloon 108 can be prevented from expanding substantially or otherwise deviating from its folded shape. In addition, the cover assembly 200 can protect the balloon 208 from scratches, tears, etc., during shipping and storage. Further, the cover assembly 200 can also facilitate pressure and/or vacuum testing of the balloon 108. More specifically, the balloon 108 can be tested in the cover assembly 200 by introduction of gas, fluid, or vacuum at the assembly location, and/or in the operating room prior to crimping of the prosthetic heart valve, because the balloon is held in its folded configuration by the contoured cavity of the cover assembly 200. Therefore, the physician does not need to perform any folding steps after balloon testing and prior to crimping the prosthetic heart valve on the balloon 108.

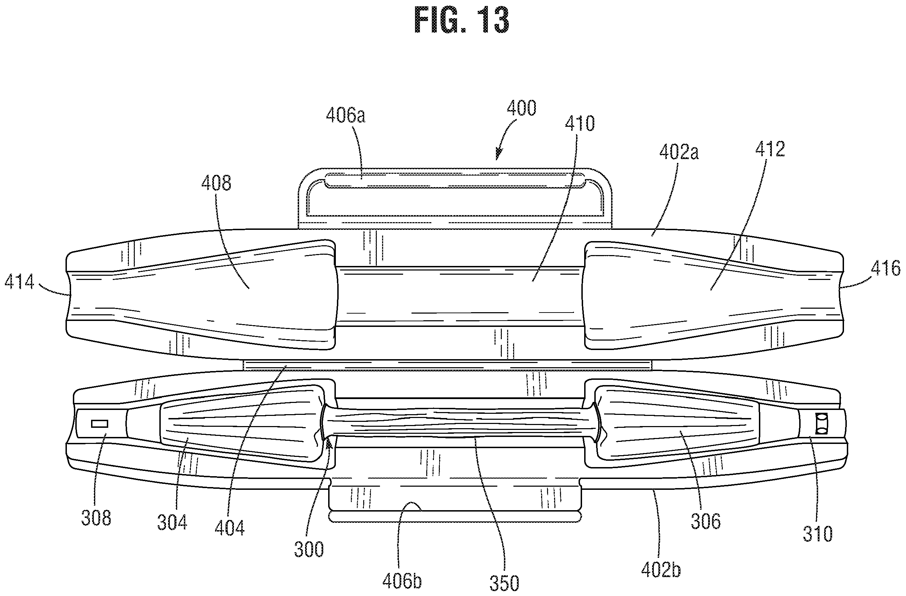

In the illustrated embodiment, the cover portions 202a, 202b are completely separable from each other. However, in other embodiments, the cover portions 202a, 202b can be connected to each other along adjacent longitudinal edges by a hinge that allows the cover portions 202a, 202b to open and close around the distal end portion 112 of the delivery device (similar to the cover 400 shown in FIG. 13, described below). In lieu of or in addition to the sleeve 204, the cover portions 202a, 202b can include complimentary latch portions that retain the cover portions in the closed state around the distal end portion 112 of the delivery device (similar to the latch portions 406a, 406b of FIG. 13, described below).

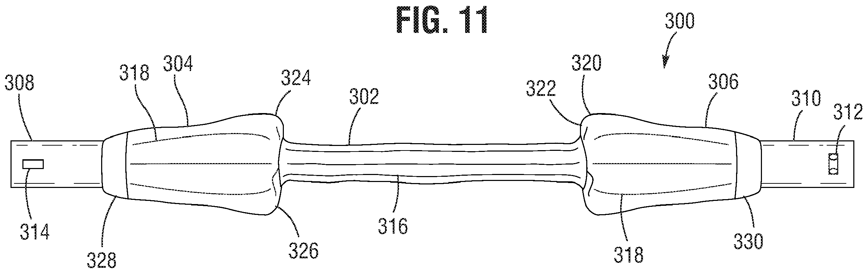

FIG. 11 shows an inflatable balloon 300 folded in a deflated state, according to another embodiment. The balloon 300 can be referred to an "off-device formed balloon," in that it can be fully formed and folded prior to being assembled on the delivery device (e.g., delivery device 100).

Similar to the balloon 108, the balloon 300 in the illustrated embodiment can include a valve retaining portion 302 having a generally cylindrical shape, a distal tapered portion 304 connected to a distal end of the valve retaining portion 302, a proximal tapered portion 306 connected to a proximal end of the valve retaining portion 302, a distal leg 308 connected to a distal end of the distal tapered portion 304, and a proximal leg 310 connected to a proximal end of the proximal tapered portion 306. The balloon 300 can be formed using techniques known in the art.

In some embodiments, the balloon 300 can include one or more markings or indicia that identify certain attributes of the balloon, such as its size, for use in assembly of the delivery device. For example, a laser marking 312 can be placed on the proximal leg 310. Likewise, an optional laser marking 314 can be placed on the distal leg 308 of the balloon. It should be understood that the markings 312 or 314 can be placed on other sections of the balloon. Also, other types of markings can be used, such as markings formed by printing, painting, or etching markings on the balloon.

The valve retaining portion 302 can include a plurality of tightly compressed axially extending folds 316. In addition, the balloon 302 can include one or more axially extending folds 318 formed along the distal tapered portion 304 and the proximal tapered portion 306.

As further shown in FIG. 11, the distal end 320 of the proximal tapered portion 306 can have a radial fold 322 which forms an obtuse or right angle relative to the valve retaining portion 302 (similar to fold 188 of FIG. 6). The proximal end 324 of the distal tapered portion 304 can have a radial fold 326 which forms an obtuse or right angle relative to the valve retaining portion 302 (similar to fold 188 of FIG. 6). The obtuse angled radial folds 322, 326 can provide a secure valve nesting zone while passing the delivery device through the loader and the introducer sheath of the delivery system.

Although not shown, the distal tapered portion 304 has two radial folds forming an S-shaped curve (similar to folds 184, 185 of FIG. 6).

As shown in FIG. 11, the distal tapered portion 304 can have a distal end section 328 that is free of any folds. Likewise, the proximal tapered portion 306 can have a proximal end section 330 that is free of any folds. As described above in connection with the embodiment of FIG. 6, the distal end section 328 creates a pleat gap that can facilitate smooth crossing of the patient's native valve during valve delivery. The proximal end section 330 creates a pleat gap that can facilitate smooth crossing of the patient's native valve during retraction of the delivery device (such as for repositioning the prosthetic valve) and retrieval of the delivery device back through the introducer sheath after valve implantation.

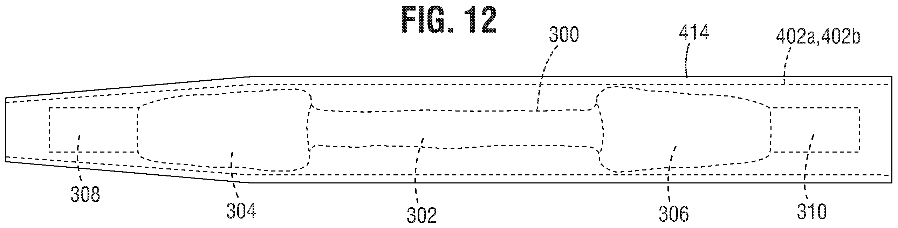

After forming the balloon 300 and created the folds, the balloon can be enclosed in a protective cover 400, as depicted in FIGS. 12-13, until it is ready to be assembled in a delivery device. Similar to cover 200, the cover 400 has an internal geometry that generally matches the shape of the folded balloon 300. In the illustrated embodiment, the cover 400 can include first and second complementary cover portions 402a, 402b, each of which is configured to receive one half of the balloon. The cover portions 402, 402b can be connected by a hinge 404 to form a clam shell configuration that allow the cover portions 402a, 402b to open and close around the balloon.

The first and second cover portions 402a, 402b can include respective latch portions 406a and 406b. When the cover portions 402a, 402b are placed around the balloon in a closed state, the latch portion 406a can engage the latch portion 406b to retain the cover portions in the closed state. In certain embodiments, the latch portion 406a can form a releasable snap fit connection with the latch portion 406b.

Each over portion 402a, 402b can have a distal recess portion 408, an intermediate recess portion 410, and a proximal recess portion 412. The distal recess portion 408 can have a shape that substantially corresponds to the shape of the distal tapered portion 304 and the distal leg 310. The intermediate recess portion 410 can have a shape that substantially corresponds to the shape of the valve retaining portion 302. The proximal recess portion 412 can have a shape that substantially corresponds to the shape of the proximal tapered portion 306 and the proximal leg 310.

In lieu of or in addition to the latch portions 406a, 406b, the cover 400 can be placed inside of a sleeve 414 (as shown in FIG. 12) to retain the cover portions 402a, 402b in the closed state around the balloon.

In alternative embodiments, the cover portions 402a, 402b can be completely separable (similar to the embodiment of FIGS. 8-10) and can be retained in the closed state by the sleeve 414 or other retaining means.

In certain embodiments, in lieu of or in addition to the cover 400, a restraining member 350, such as in the form of a sleeve, can be placed around the valve retaining portion 302 of the balloon. The restraining member 350 can assist in retaining the valve retaining portion 302 of the balloon in a compressed and folded state until the balloon 300 is assembled onto a delivery device, as described below. The restraining member 350 can be formed from a relatively thin sheet of polymeric material, such as polyester, polyurethane, or fluorinated ethylene propylene (FEP). In some embodiments, the restraining member 350 can be removed by the manufacturer after assembly of the delivery device. In other embodiments, the restraining member can be kept in place after assembly and removed by an end user prior to crimping a prosthetic valve on the balloon. Moreover, it should be understood that the restraining member 350 can be used with other embodiments disclosed herein, such as the balloon 108. In other embodiments, the restraining member 350 is not used and only the cover 400 is used to retain the valve retaining portion 302 in its compressed and folded state.

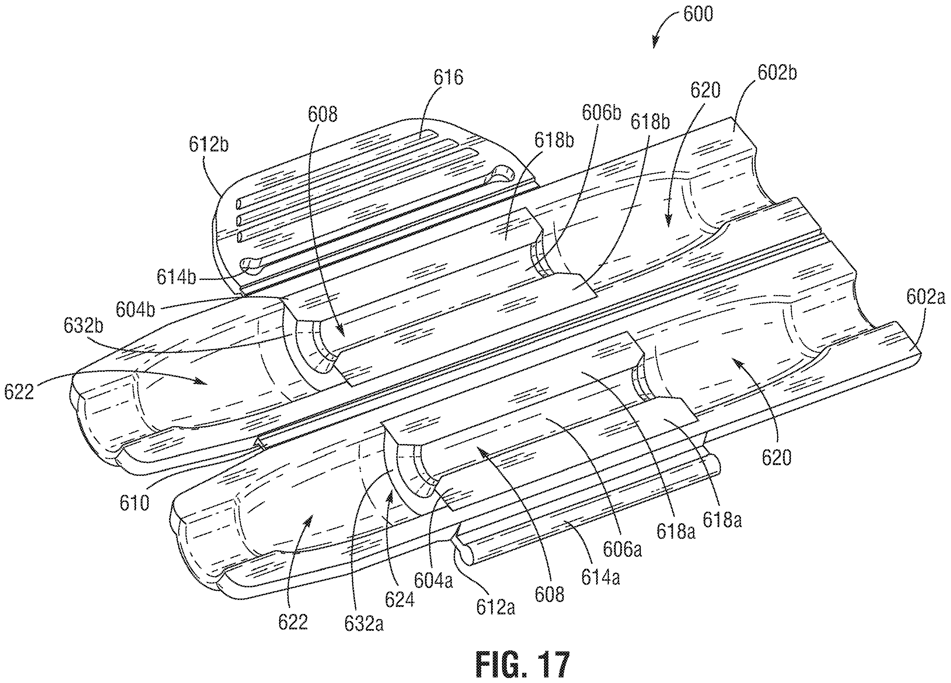

FIGS. 17-19 show another balloon cover 600 for a folded balloon 680 mounted on a delivery catheter 670, according to an alternative embodiment.

As described herein, the balloon 680 can be any of the balloons 108, 208, 300 disclosed above or any other types of foldable balloons, and the delivery catheter 670 can be the same delivery devices 100, 500 described above, or any other types of delivery devices for implanting a balloon-inflatable prosthetic valve, as known in the art.

For example, in the depicted embodiment, the delivery device 670 can include an outer shaft 672, an inner shaft 674 extending through a central lumen of the outer shaft 672, and a nose cone 676 coupled to the inner shaft 674. The balloon 680 can be supported on a shoulder assembly of the delivery device 670. The shoulder assembly can include a proximal shoulder 690 connected to a distal end of the outer shaft 672 and a distal shoulder 692 mounted on the inner shaft 674. The balloon 680 can have a proximal end portion 682 surrounding and/or folded over the proximal shoulder 690, a distal end portion 684 surrounding and/or folded over the distal shoulder 692, and a valve retaining portion 686 located between the proximal end portion 682 and the distal end portion 684. The proximal end portion 682 of the balloon 680 may be secured to the distal end portion of the outer shaft 672. The distal end portion 684 of the balloon 680 may be secured to an outer surface of the nose cone 676.

As shown, the balloon cover 600 can include an outer shell 602 and an inner sleeve 604 disposed within the outer shell 602. The inner sleeve 604 can have an inner surface 606 defining a lumen 608. In an example embodiment, the lumen 608 is configured to receive the valve retaining portion 686 of the balloon 680.

Prior to crimping a prosthetic valve on the balloon of a delivery device, the user typically performs a cyclic "de-airing" process that involves pushing inflation fluid into the balloon and then withdrawing the fluid out of the balloon, such as with a syringe fluidly connected to the handle of the delivery device. The de-airing process can be more effective when the balloon is allowed to at least partially inflate. However, inflation of the balloon outside of a balloon cover can result in un-folding of the balloon, which can inhibit or prevent the balloon from returning to its folded state when the inflation fluid is removed from the balloon. As described more fully below, the inner sleeve 604 can be deformable under pressure exerted against the inner surface 606 by the balloon 680 upon inflation of the balloon 680, thereby radially expanding the diameter of the lumen 608. This allows at least partial inflation of the balloon while performing the de-airing procedure while the balloon is contained within the cover. During the de-airing process, the deformable inner sleeve 604 can prevent complete unfolding of the balloon and/or assist the balloon in returning to its fully folded state after the inflation fluid is removed from the balloon.

In some embodiments, the outer shell 602 can be more rigid than the inner sleeve 604. For example, the inner sleeve 604 can be made of an elastomer such as natural rubbers, styrene-butadiene block copolymers, polyisoprene, polybutadiene, ethylene propylene rubber, ethylene propylene diene rubber, silicone elastomers, fluoroelastomers, polyurethane elastomers, nitrile rubbers, or a mixture of these materials. The inner sleeve 604 can also include a shape memory material as described further below. The outer shell 602 can be made of a non-deformable material such as polyvinyl chloride, glass, poly(methyl methacrylate), acrylonitrile butadiene styrene, styrene acrylonitrile, polystyrene, silicone, cellulose, polyester, polyolefins, polyethylene, metal, ceramic, or a mixture of these materials.

In the depicted embodiments, the outer shell 602 includes a first shell member 602a and a second shell member 602b. The first and second shell members 602a, 602b can be connected by a hinge 610 forming a clam shell configuration that allows the shell members 602a, 602b to open and close around the balloon 680.

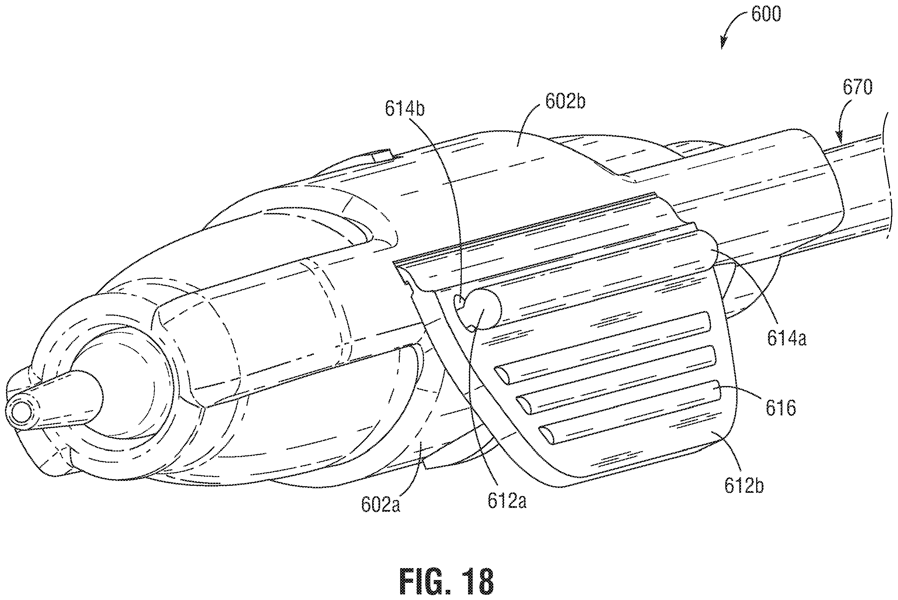

In certain embodiments, the first and second shell members 602a, 602b can include respective latch portions. When the first and second shell members 602a, 602b are placed around the balloon 680 and the inner sleeve 604 in a closed state, the respective latch portions can engage each other, as illustrated in FIG. 18, to retain the shell members 602a, 602b in the closed state.

For example, as best shown in FIG. 17, one latch portion can be configured as a ridge 612a extending outwardly from the first shell member 602a, and another latch portion can be configured as a cantilevered tab 612b extending outwardly from the second shell member 602b. The ridge 612a can have an enlarged rim or edge portion 614a along its free longitudinal edge, and the tab 612b can have a corresponding slot 614b. The slot 614b can be so positioned and sized as to allow the enlarged rim 614a to extend through to form a snap-fit when the shell members 602a, 602b are placed around the balloon 680 and the inner sleeve 604 in a closed state.

In some embodiments, the width of the slot 614b can be slightly smaller than the width of the enlarged rim 614a and the tab around the slot 614b can be made of an elastic or deformable material. Thus, by pressing the ridge 612a and the tab 612b against each other, the slot 614b can enlarge slightly to allow the enlarged rim 614a to extend therethrough and then revert back to its original size and shape after the rim 614a passes through the slot 614b. The narrower slot 614b relative to the enlarged rim 614a prevents the rim 614a from inadvertently sliding back through the slot 614b in the opposite direction, thus ensuring a tight interlocking between the ridge 612a and the tab 612b. To open the shell members 602a, 602b, a user can separate the ridge 612a from the tab 612b, e.g., by manually pulling the enlarged rim 614a out of the slot 614b. In some embodiments, the tab 612b can have a plurality of bumps and/or indents 616 located on its surface so as to provide friction points for a user's finger to firmly grab the tab 612b for engaging or disengaging the ridge 612a and the tab 612b.

It should be understood that the latching mechanism described above is merely an example embodiment. Other latching mechanisms that are known in the art can also be used to couple and decouple between the shell members 602a, 602b, such as hook-and-loop fasteners, clips, buckles, magnetic fasteners, etc.

In the depicted embodiment, when the shell members 602a, 602b are in the closed state, outer shell 602 can define a proximal recess 620, a distal recess 622, and an intermediate recess 624 located between the proximal recess 620 and the distal recess 622.

The proximal recess 620 can have a tapered shape such that a proximal end of the proximal recess 620 has a smaller diameter than a distal end of the proximal recess 620. In certain embodiments, the shape of the proximal recess 620 can substantially correspond to the shape of the proximal end portion 682 of the balloon 680.

The distal recess 622 can have a tapered shape such that a distal end of the distal recess 622 has a smaller diameter than a proximal end of the distal recess 622. In some embodiments, the shape of the distal recess 622 can substantially correspond to the shape of the distal end portion 684 of the balloon 680.

The intermediate recess 624 can have a generally cylindrical shape that is configured to receive the inner sleeve 604.

In the depicted embodiment, the shell members 602a, 602b have about the same circumferential size such that each defines a half section of the proximal recess 620, the distal recess 622, and the intermediate recess 624. In other embodiments, the shell members 602a, 602b can have different circumferential sizes such that one of the shell members defines a smaller section whereas the other shell member defines the remaining (i.e., a larger) section of the recesses 620, 622, and 624.

In the depicted embodiment, the inner sleeve 604 has first and second portions 604a, 604b. Each portion has a semi-cylindrical interior surface 606a or 606b defining a half section of the lumen 606.

In other embodiments, the first and second portions 604a, 604b can have different circumferential sizes such that one portion defines a less-than-half section whereas the other portion defines the remaining (i.e., larger-than-half) section of the lumen 608.

In the depicted embodiment, the first portion 604a of the inner sleeve 604 has two longitudinal edges 618a that are configured to matingly engage two respective longitudinal edges 618b of the second portion 604b of the sleeve 604 to form the lumen 606. Thus, when the longitudinal edges 618a of the first portion 604a are aligned with the respective longitudinal edges 618b of the second portion 604b, the inner sleeve 604 can enclose the valve retaining portion 686 of the balloon 680 in a closed state.

In some embodiments, the first and second portions 604a, 604b are completely separable at both pairs of longitudinal edges 618a, 618b. In other embodiments, the first and second portions 604a, 604b can be hingely connected along one pair of longitudinal edges 618a, 618b while they can be separable at the other pair of longitudinal edges 618a, 618b.

As described herein, the inner sleeve 604 can be radially deformable when the balloon 680 is inflated and exerts pressure radially outwardly against the inner surface 606 of the inner sleeve 604, such as during a de-airing procedure. In other words, inflating the balloon 680 from a deflated state to an inflated state can deform the lumen 606 of the inner sleeve 604 from an initial state to a deformed state. The lumen 608 can have a first diameter in the initial state and a second diameter in the deformed state, and the second diameter is larger than the first diameter.

In the depicted embodiment, the inner surface 606 of the inner sleeve 604 can maintain enclosure of the lumen 608 in a circumferentially continuous manner when the lumen 608 is radially expanded by inflating the balloon 680 from the deflated state to the inflated state. In other words, the inner surface 606 can be circumferentially continuous where there is no gap between either engaging pairs of longitudinal edges 618a, 618b through which a portion of the balloon 680 can extend. Thus, the two semi-cylindrical interior surfaces 606a or 606b can form a full cylindrical interior surface that defines the lumen 608 irrespective of the lumen's diameter.

In some embodiments, the inner sleeve 604 can further define a tapered proximal opening 626 located at a proximal end of the lumen 608 and a tapered distal opening 628 located at a distal end of the lumen 608. The tapered proximal opening 626 connects the middle portion 630 of the lumen 608 to the proximal recess 620 and the tapered distal opening 628 connects the middle portion 630 of the lumen 608 to the distal recess 622.

In some embodiments, at least when the balloon 680 is in the deflated state (i.e., the lumen 608 is in the initial state), the middle portion 630 of the lumen 608 can have a diameter that is smaller than both the tapered proximal opening 626 and the tapered distal opening 628.

In some embodiments, the tapered proximal opening 626 can be configured to receive a junctional region of the balloon 680 where the valve retaining portion 686 connects with the proximal end portion 682, and the tapered distal opening 628 can be configured to receive a junctional region of the balloon 680 where the valve retaining portion 686 connects with the distal end portion 684.

In some embodiments, the axial length of the inner sleeve 604 is configured to be about the same as the axial length of the intermediate recess 624 of the outer shell 602 so that the inner sleeve 604 and the valve retaining portion 686 of the balloon 680 can snuggly fit into the intermediate recess 624. In some embodiments, the axial length of the inner sleeve 604 is configured to remain fixed when inflating the balloon 680 from the deflated state to the inflated state. Thus, the inner sleeve 604 and the valve retaining portion 686 can remain snuggly fit inside the intermediate recess 624 regardless of whether the balloon 680 is in a deflated or inflated state.

In an example embodiment, the inner sleeve 604 includes an inner wall portion 632 that surrounds the lumen 608 and one or more legs 634 extending radially outwardly from the inner wall portion 632 and spaced apart axially from each other along the length of the inner wall portion 632. Specifically, the inner wall portion 632 can be divided into two half wall portions 632a, 632b which respectively define the semi-cylindrical interior surfaces 606a and 606b of the first and second separable portions 604a, 604b. When both the inner sleeve 604 and the outer shell 602 are in the closed state, the one or more legs 634 can press against an inner surface 636 of the outer shell 602 so as to form one or more cavities 638 between the inner wall portion 632 of the inner sleeve 604 and the inner surface 636 of the outer shell 602.

In the depicted example, the inner sleeve 604 has seven legs 634 which divides the space between the inner wall portion 632 and the inner surface 636 into six cavities 638. In other embodiments, the number (N) of legs can be less than 7 (e.g., 2, 3, 4, 5, 6) or more than 7 (e.g., 8, 9, or more), and the number of cavities can be N-1.

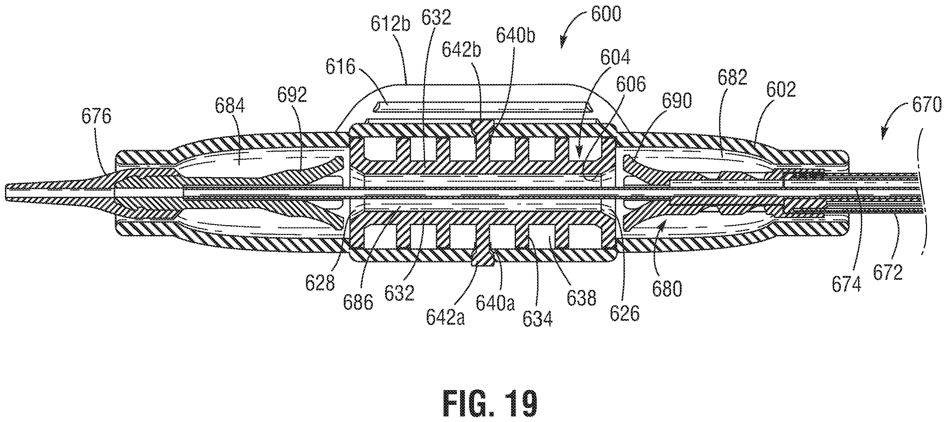

As illustrated in FIG. 19, the one or more legs 634 can be parallel to each other. In some embodiments, each of the one or more legs 634 can extend circumferentially around the inner wall portion 632.

For example, each leg 634 can be divided into a pair of half legs that are respectively located on the two half wall portions 632a, 632b. Each of the half legs can extend circumferentially around the corresponding half wall portion 632a or 632b for about 180 degrees so that when the two half wall portions 632a, 632b are aligned together to enclose the lumen 608, the pair of half legs abut each other to form a complete leg 634 that completely encircles the inner wall portion 632 (i.e., the leg 634 extends circumferentially around the inner wall portion 632 for 360 degrees). As such, a cavity between two adjacent, complete legs can be a closed space that is isolated from any of its neighboring cavities.

In other embodiments, some of the legs 634 may not fully encircle the inner wall portion 632. For example, a leg may extend circumferentially along one or more discontinuous segments of the inner wall portion 632 such that two cavities on opposite sides of the leg are connected to each other through a gap in the leg.

In some embodiments, the one or more legs 634 can be compressible in the radial direction. For example, when inflating the balloon 680 from the deflated state to the inflated state, the lumen 608 can be radially expanded from the initial state (with a relatively smaller diameter) to the deformed state (with a relatively larger diameter). Correspondingly, the legs 634 can be compressed from an initial radial length to a shortened radial length. Thus, the space occupied by the cavities 638 between the legs 634 can be reduced from an initial volume to a reduced volume.

Conversely, when deflating the balloon 680 from the inflated state to the deflated state, the lumen 608 can revert back from the deformed state to the initial state. For example, the legs 634 can be made of an elastomer or a shape memory material, including any of various polymers or alloys. Thus, the legs 634 can recover their initial radial length after the radial pressure exerted by the inflated balloon is removed. Correspondingly, the space of each cavities 638 between the legs 634 can revert back to its initial volume. Note that the contracting lumen 608 can press inwardly against the balloon 680 and help prevent the folded balloon 680 (in deflated state) from unfolding.

In some embodiments, the inner surface 636 of the outer shell 602 can have a receptacle configured to receive at least one of the legs 634 so as to securely couple the inner sleeve 604 to the outer shell 602.

For example, as depicted in FIG. 19, the first and second shell members 602a, 602b can have a pair of receptacles 640a, 640b, such as in the form of openings in the shell members 602a, 602b, located at the center (along the axial direction) of their respective inner surface. A pair of half legs located at the center (along the axial direction) of the two half wall portions 632a, 632b (which can be joined together to form the middle leg) can have enlarged foot portions 642a, 642b. The pair of receptacles 640a, 640b can be configured to matingly receive the respective foot portions 642a, 642b so that the first and second separable portions 604a, 604b of the inner sleeve can be securely coupled to the respective first and second shell members 602a, 602b.

In some embodiments, the receptacle and the coupling leg can be located off the center (along the axial direction) of the balloon cover. In some embodiments, a plurality of receptacles can be spaced apart on the outer shell 602 along the axial direction, and each receptacle can be configured to matingly receive a corresponding leg of the inner sleeve 604. In some embodiments, the inner surface 636 of the outer shell 602 can have one or more protrusions that are configured to matingly engage corresponding one or more receptacles located on the legs 634 of the inner sleeve 604. In some embodiments, the legs 634 of the inner sleeve 604 and the inner surface 636 of the outer shell 602 can be securely coupled together by other mechanisms, such as gluing, tongue-and-groove joints, clips, buckles, magnetic fasteners, etc.

In some embodiments, the inner sleeve 604 and the outer shell 602 can be removably coupled together (i.e., they can be separated apart). In other embodiments, the inner sleeve 604 and the outer shell 602 can be fixedly coupled together (i.e., the inner sleeve 604 cannot be removed from the outer shell 602).

In other embodiments, the inner sleeve 604 can include only the inner wall portion 632 without the legs 634. For example, the inner wall portion 632 can be made of a compressible or viscoelastic material such as polyurethane foam, latex, or other types of shape memory foams, or superelastic alloys that have shape memory properties. Thus, when inflating the balloon 680 from the deflated state to the inflated state, the inflating force of the balloon 680 can compress the inner wall portion 632 and reduce its thickness, thus increasing the diameter of the lumen 608. Conversely, when deflating the balloon 680 from the inflated state to the deflated state, the inner wall portion 632 can revert back to its original shape with a larger wall thickness, thus decreasing the diameter of the lumen 608. Thus, the contracting lumen 608 can press inwardly against the balloon 680 and help retain the deflated balloon 680 in its folded shape.

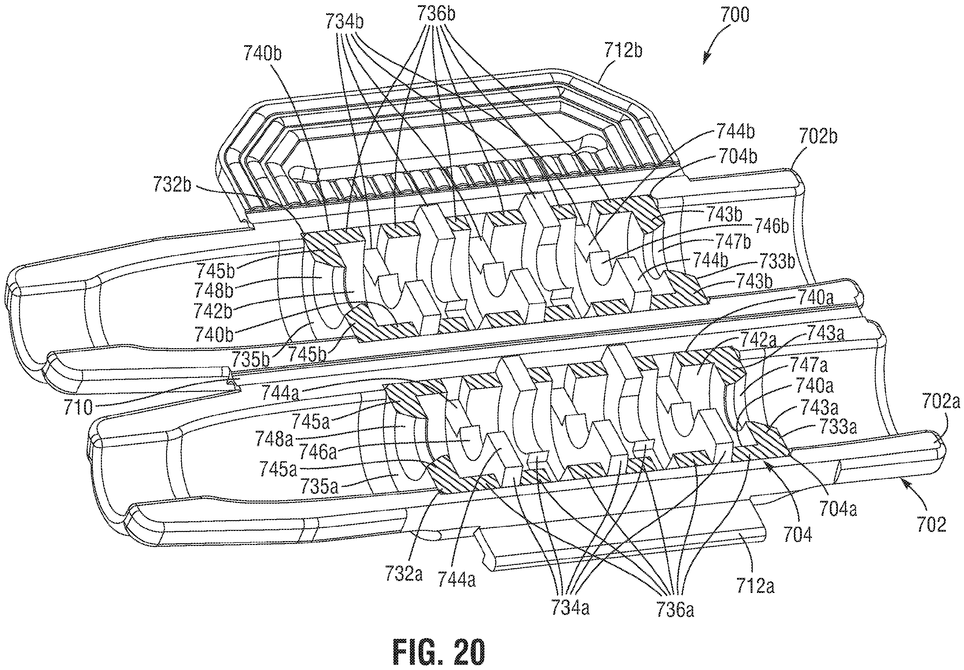

FIGS. 20-21 show another balloon cover 700, according to an alternative embodiment.

Similar to the balloon cover 600, the balloon cover 700 can have an outer shell 702 and an inner sleeve 704 disposed within the outer shell 702, and the outer shell 702 can be more rigid than the inner sleeve 704. The outer shell 702 can include a first shell member 702a and a second shell member 702b, which are connected to each other by a hinge 710. Thus, the shell members 702a, 702b can form a clam shell configuration, allowing them to open and close around a balloon mounted on a delivery catheter (not shown). Likewise, the first and second shell members 702a, 702b can include respective latch portions 712a, 712b, which can be configured to engage each other to retain the shell members 702a, 702b in the closed state.

As shown in FIG. 20, the inner sleeve 704 can have first and second separable sleeve portions 704a, 704b. The first sleeve portion 704a includes a wall portion 732a and five rib portions 734a extending radially inwardly from the wall portion 732a, and the second sleeve portion 704b includes a wall portion 732b and five rib portions 734b extending radially inwardly from the wall portion 732b.

Although each of the first and second sleeve portions 704a, 704b has five rib portions, it should be understood that the number of rib portions in each sleeve portion can be less than five (e.g., from one to four) or more than five, and the same principles described herein apply.

In some embodiments, the rib portions 734a, 734b are distributed uniformly along the longitudinal axis of the respective sleeve portions 704a, 704b. In other embodiments, the rib portions 734a, 734b can be distributed non-uniformly along the longitudinal axis of the respective sleeve portions 704a, 704b.

The wall portion 732a includes a plurality of wall segments 736a separated by the rib portions 734a, and the wall portion 732b includes a plurality of wall segments 736b separated by the rib portions 734b. The number of wall segments in each sleeve portion can be the number of rib portions on the sleeve portion plus one (six in the depicted example).

Each wall segment 736a of the first sleeve portion 704a has two longitudinal edges 740a and a wall surface 742a extending between the two longitudinal edges 740a. Similarly, each wall segment 736b of the second sleeve portion 704b has two longitudinal edges 740b and a wall surface 742b extending between the two longitudinal edges 740b.

Each rib portion 734a of the first sleeve portion 704a has two radial edges 744a and a rib surface 746a extending between the two radial edges 744a. Similarly, each rib portion 734b of the second sleeve portion 704b has two radial edges 744b and a rib surface 746b extending between the two radial edges 744b.

In addition, the first sleeve portion 704a includes a proximal end plate 733a and a distal end plate 735a that are generally parallel to the rib portions 734a, and the second sleeve portion 704b includes a proximal end plate 733b and a distal end plate 735b that are generally parallel to the rib portions 734b. The proximal end plate 733a has a pair of radial edges 743a and an inner surface 747a extending between the pair of radial edges 743a. The distal end plate 735a has a pair of radial edges 745a and an inner surface 748a extending between the pair of radial edges 745a. Similarly, the proximal end plate 733b has a pair of radial edges 743b and an inner surface 747b extending between the pair of radial edges 743b. The distal end plate 73 5b has a pair of radial edges 745b and an inner surface 748b extending between the pair of radial edges 745b.

As described herein, the first and second sleeve portions 704a, 704b can be matingly coupled together so that the inner sleeve 704 is in a closed state around the balloon.

For example, the two radial edges 744a of each rib portion 734a of the first sleeve portion 704a can be configured to matingly engage (e.g., through an interlocking mechanism) two radial edges 734b of a corresponding rib portion 734b of the second sleeve portion 704b so that the rib surfaces 746a of the first sleeve portion 704a can adjoin corresponding rib surfaces 746b of the second sleeve portion 704b to form five rib enclosures 750. In other words, five rib enclosures 750 can be formed by matingly engaging five respective pairs of rib portions 734a, 734b. In some embodiments, all rib enclosures 750 have about the same diameter.

In addition, the two longitudinal edges 740a of each wall segment 736a of the first sleeve portion 704a can be configured to align with or matingly engage two longitudinal edges 740b of a corresponding wall segment 736b of the second sleeve portion 704b so that the wall surfaces 742a of the first sleeve portion 704a can adjoin corresponding wall surfaces 742b of the second sleeve portion 704b to form six wall enclosures 752 separated by five pairs of rib portions 734a, 734b. In some embodiments, all wall enclosures 752 have about the same diameter.

Further, the pair of radial edges 743a of the proximal end plate 733a can be configured to align with or matingly engage the pair of radial edges 743b of the proximal end plate 733b so that the inner surfaces 747a, 747b of the proximal end plates 733a, 733b can be adjoined to form a proximal opening 726. Similarly, the pair of radial edges 745a of the distal end plate 73 5a can be configured to align with or matingly engage the pair of radial edges 745b of the distal end plate 735b so that the inner surfaces 748a, 748b of the distal end plates 735a, 735b can be adjoined to form a distal opening 728. In some embodiments, the proximal and distal openings 726, 728 can have tapered shapes similar to the proximal and distal openings 626, 628 described above.

In some embodiments, the rib enclosures 750 can define a lumen which is configured to receive the valve retaining portion (e.g., 686) of the balloon (e.g., 680). For example, when the first and second sleeve portions 704a, 704b are matingly coupled together, all rib enclosures 750 can have about the same diameter, which is about the same as the diameter of the valve retaining portion of the balloon in its compressed or folded state. Thus, the valve retaining portion of the folded balloon can be retained within the plurality of rib enclosures 750, which are spaced apart from each other and distributed along the length of the inner sleeve 704.

In some embodiments, when inflating the balloon retained within the rib enclosures 750, the inner sleeve 704 can be deformed under pressure exerted against the rib surfaces 746a, 746b by the balloon, thereby radially expanding the diameter of the rib enclosures 750.

For example, the first and second sleeve portions 704a, 704b can further include a plurality of legs (not shown) extending radially outwardly from the wall portions 732a, 732b to the inner surface of respective outer shell members 702a, 702b. Such legs can be compressible in the radial direction similar to the legs 634 described above. Thus, when inflating the balloon from the deflated state to the inflated state, the rib enclosures 750 can be radially expanded from the initial state (with a relatively smaller diameter) to the deformed state (with a relatively larger diameter) while the legs are compressed from an initial radial length to a shortened radial length. Conversely, when deflating the balloon from the inflated state to the deflated state, the rib enclosures 750 can revert back from the deformed state to the initial state while the legs recover to their initial radial length after the radial pressure exerted by the inflated balloon is removed.

Alternatively, the rib portions 734a, 734b can be made of a compressible or viscoelastic material having shape memory properties. Thus, when inflating the balloon from the deflated state to the inflated state, the inflation force of the balloon can radially compress the rib portions 734a, 734b, thus increasing the diameter of the rib enclosures 750. Conversely, when deflating the balloon from the inflated state to the deflated state, the rib portions 734a, 734b can revert back to their original shape, thus decreasing the diameter of the rib enclosures 750.

In some embodiments, inflating the balloon not only can increase the diameter of the rib enclosures 750, but also can transmit the force to the diameter of the wall segments 736a, 736b and cause corresponding increase the diameter of the wall enclosures 752. Conversely, when the balloon is deflated, both the rib enclosures 750 and the wall enclosures 752 can return to their original (smaller) diameters.

In other embodiments, inflating the balloon can increase the diameter of the rib enclosures 750 without changing the diameter of the wall enclosures 752. This can occur, for example, when the rib portions 734a, 734b are made of a compressible or viscoelastic material while the wall segments 736a, 736b are made of a more rigid material.

Generally, the diameter of the wall enclosures 752 is larger than the diameter of the rib enclosures 750, regardless the balloon is in the deflated state or inflated state. In some embodiments, when the balloon is in the inflated state, while sections of the balloon are retained within the rib enclosures 750, portions of the balloon outside the rib enclosures 750 may partially bulge into the wall enclosures 752.

In some embodiments, any two adjacent rib portions can be configured to have a circumferential offset such that the two radial edges of one of the two adjacent rib portions extend angularly relative to respective two radial edges of the other adjacent rib portion.

For example, as shown in FIG. 20, the five rib portions 734a of the first sleeve portion 704a are configured to have alternate rotational positions such that the corresponding radial edges of the first, third, and fifth rib portions (numbering from the most distal one to the most proximal one) extend in a first angular direction, whereas the corresponding radial edges of the second and fourth rib portions extend in a second angular direction that has a circumferential offset relative to the first angular direction. The five rib portions 734b of the second sleeve portion 704b have similar alternate rotational positions.

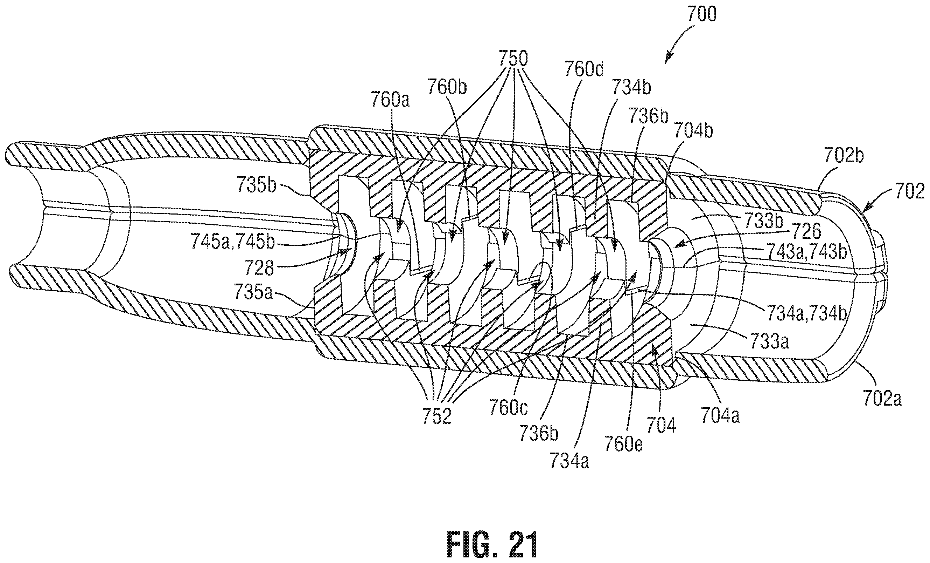

FIG. 21 shows a cross-section of the cover 700 when the first and second sleeve portions 704a, 704b are in a closed configuration. The locations where adjacent radial edges 734a, 734b mate with each other form parting lines 760a, 760b, 760c, 760d, 760e. As shown, each parting line 760a-e is circumferentially offset from an adjacent parting line with respect to a central longitudinal axis of the cover 700. The offset between the parting lines minimizes the risk of the balloon extending outwardly through the parting lines when the balloon in inflated during use.

In some embodiments, the plurality of rib portions do not have alternate rotational orientations. For example, the plurality of rib portions on a sleeve portion can be configured to rotate clockwise (or counter-clockwise) progressively from the most distal rib portion to the most proximal rib portion. In other embodiments, the plurality of rib portions on a sleeve portion can be randomly rotated or rotated in a predefined pattern so that no two adjacent rib portions have radial edges extending in the same angular direction.

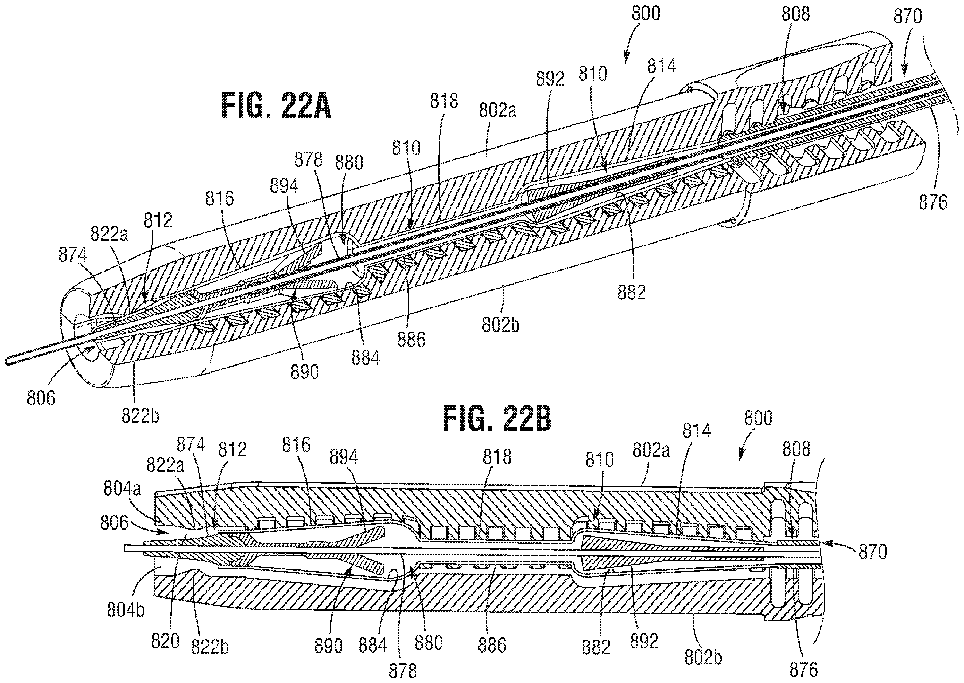

FIGS. 22-23 show another balloon cover 800 configured to receive a delivery device, such as a delivery device 870, according to yet another embodiment.

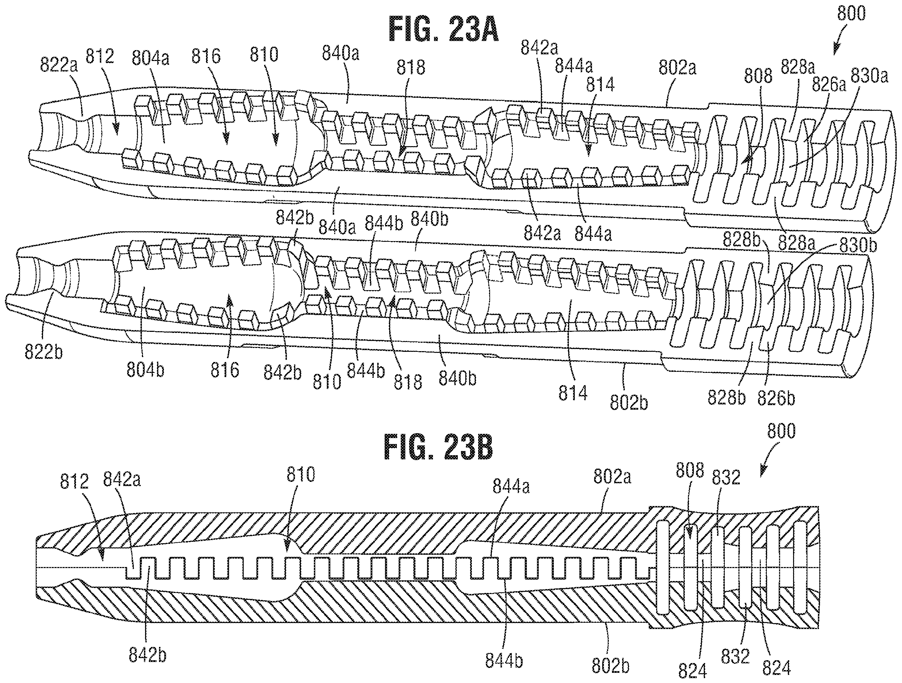

As shown, the balloon cover 800 includes a first shell member 802a and a second shell member 802b that are configured to matingly engage each other. When the first and second shell members 802a, 802b are mating engaged with each other, the balloon cover 800 is in a closed state, as shown in FIGS. 22B and 23B. The respective inner surfaces (or "inner walls") 804a, 804b of the shell members 802a, 802b can define a lumen 806 that is configured to receive a distal end portion of the balloon catheter 870, including an inflatable balloon 880 mounted thereto. When the shell members 802a, 802b are disengaged from each other, the balloon cover 800 is in an open state, as shown in FIG. 23A.

In the depicted embodiment, the delivery device 870 includes an outer shaft (not shown), an intermediate shaft 876 (also referred to as a "balloon shaft") extends through a lumen of the outer shaft, an inner shaft 878 extends through a lumen of the balloon shaft 876, and a nosecone 874 connected to the distal end portion of the inner shaft 878. Similar to the examples described above (see e.g., FIGS. 6-7), the delivery device 870 can include a shoulder assembly 890 connected to the distal end portion of balloon shaft 876, and the balloon 880 can be folded onto the shoulder assembly 890.

In the depicted embodiment, the first shell member 802a has two opposing first longitudinal edges 840a and the second shell member 802b has two opposing second longitudinal edges 840b. The first longitudinal edges 840a can be configured to mate with the corresponding second longitudinal edges 840b to form two longitudinal parting lines.

In some embodiments, the shell members 802a, 802b can be completely separable from each other, i.e., both first longitudinal edges 840a can be separated from respective longitudinal edges 840b (see e.g., FIG. 23A). In alternative embodiments, the first and second shell members 802a, 802b can be connected to each other along one (and only one) pair of longitudinal edges 840a, 840b by a hinge (similar to the hinges 610 or 710 described above), thereby allowing the first and second shell members 802a, 802b to open and close around the distal end portion of the balloon catheter 870.

In certain embodiments, the first and second shell members 802a, 802b can have respective latches (similar to the latches 406a and 406b, or 612a and 612b described above) which are configured to engage each other so as to retain the first and second shell members 802a, 802b matingly engaged (i.e., in the closed state).

In other embodiments, the balloon cover 800 can further include a sleeve (not shown, similar to the sleeve 204 described above) configured to slide over the first and second shell members 802a, 802b so as to retain the first and second shell members matingly engaged (i.e., in the closed state).

In the depicted embodiment, the lumen 806 can include a proximal shaft section 808 configured to receive a distal end portion of the intermediate shaft 876 of the delivery device 870, a distal nosecone section 812 configured to receive the nosecone 874 of the balloon catheter 870, and an intermediate balloon section 810 configured to receive the balloon 880 mounted on the shoulder assembly 890 of the delivery device 870. The balloon section 810 is located between the shaft section 808 and the nosecone section 812.

In another embodiment, the balloon cover 800 can be configured to have a lumen that only includes the balloon section 810 without the shaft section 808 or nosecone section 812. In yet an alternative embodiment, the balloon cover 800 can be configured to have a lumen that includes only the balloon section 810 and one of the shaft section 808 and nosecone section 812.

As shown, the balloon section 810 can include a proximal shoulder portion 814 (also referred to as the "proximal compartment") configured receive a proximal portion 882 of the balloon 880 mounted on a proximal shoulder 892 of the shoulder assembly 890, a distal shoulder portion 816 (also referred to as the "distal compartment") configured to receive a distal portion 884 of the balloon 880 mounted on a distal shoulder 894 of the shoulder assembly 890, and an intermediate portion 818 (also referred to as the "intermediate compartment") between the proximal and distal shoulder portions 814, 816. The intermediate portion 818 can be configured to receive a valve retaining portion 886 of the balloon 880 located between the proximal and distal portions 882, 884 of the balloon.

Generally, except for the wedges and cavities as described below, the proximal shoulder portion 814 of the lumen can have a shape that substantially corresponds to the outer contour of the proximal portion 882 of the balloon folded on the proximal shoulder 892. Similarly, the distal shoulder portion 816 of the lumen can have a shape that substantially corresponds to the outer contour of the distal portion 884 of the balloon folded on the distal shoulder 894, and the intermediate portion 818 of the lumen can have a shape that substantially corresponds to the outer contour of the folded valve retaining portion 886 of the balloon. Specifically, in the depicted embodiment, the proximal shoulder portion 814 tapers radially outwardly from its proximal end to its distal end, and the distal shoulder portion 816 tapers radially outwardly from its distal end to its proximal end. The intermediate portion 818 has a generally cylindrical shape. The intermediate portion 818 has a smaller diameter than the proximal end of distal shoulder portion 814 and the distal end of the proximal shoulder portion 816.

Generally, the diameter of the lumen can vary along its axial length. For example, as described above, the balloon portions 882, 884 folded around the respective shoulders 892, 894 can have respective tapered shape and the valve retaining portion 886 can have a smaller diameter than the balloon portions 882, 884. Accordingly, the intermediate portion 818 of the lumen can have a smaller diameter than a distal end portion of the proximal shoulder portion 814 and a proximal end portion of the distal shoulder portion 816.

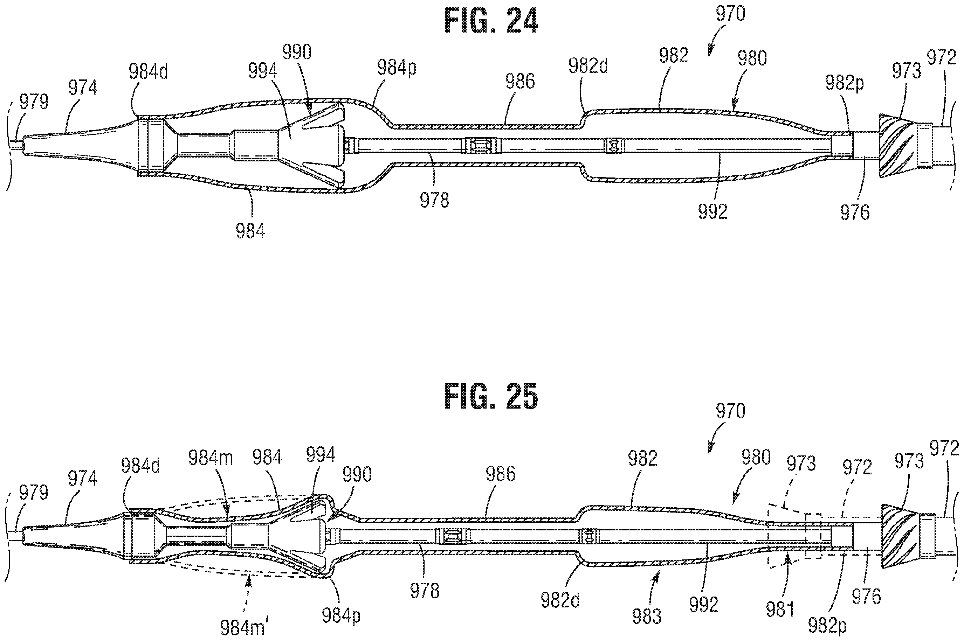

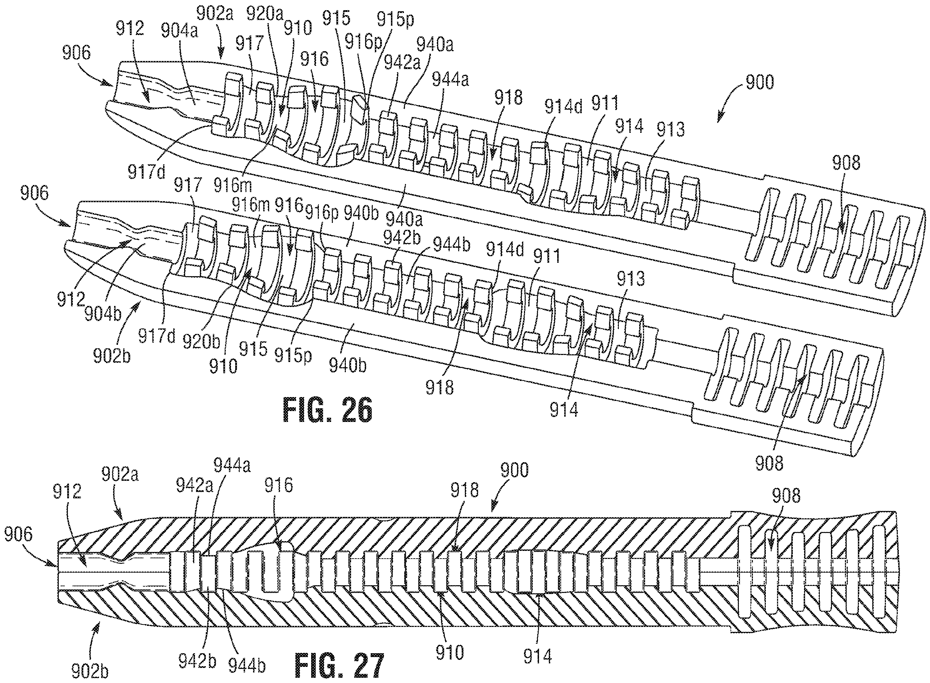

In another embodiments, the shoulder assembly 890 of the delivery device 870 includes the distal shoulder 894 but has no proximal shoulder 892. In such circumstances, while the distal portion 884 of the balloon 880 can be mounted on the distal shoulder 894 of the shoulder assembly 890, the proximal portion 882 of the balloon 880 can be mounted on the distal end of the intermediate shaft 876, similar to the embodiments shown in FIGS. 24-25 below. The portion 814 of the balloon section 810 of the lumen defined by the balloon cover 800 can still be configured to receive the proximal portion 882 of the balloon 880 (despite the absence of proximal shoulder 892), similar to the embodiment shown in FIG. 27 below.

In some embodiments, the nosecone section 812 of the lumen can have a neck portion 820. The neck portion 820 can be formed by two matching semi-annular rings 822a, 822b protruding radially inwardly from the respective inner surfaces 804a, 804b of the first and second shell members, Thus, when the balloon 800 is in the closed state, the two semi-annular rings 822a, 822b can form neck portion 820 that has a smaller diameter than the remaining portions of the nosecone section 812. By constraining the space at the distal end portion of the lumen, the narrower neck portion 820 can help maintain the folded shape of the balloon 880.

In some embodiments, the shaft section 808 of the lumen can include a plurality of central passages or apertures 824. Each central passage can be formed by a corresponding pair of opposing rib members 826a, 826b extending radially inwardly from respective inner surfaces 804a, 804b of the first and second shell members 802a, 802b. Each rib member 826a (or 826b) can have two radial edges 828a (or 828b) and a semicircular rib surface 830a (or 830b) extending between the two radial edges 828a (or 828b) such that when the balloon 800 is in the closed state, the two rib surfaces 830a, 830b can jointly define an inner surface of the respective central passage 824. As shown, any two adjacent central passages 824 can be separated by a chamber 832 located between the rib members 826a, 826b that form the two adjacent central passages 824.

Generally, the diameter of the chamber 832 (measured across the inner surface 804a, 804b) is larger than the diameter of the central passages 824 (measured across the rib surfaces 830a, 830b). In some embodiments, the plurality of passages 824 can have varying diameters (e.g., in FIG. 23B, the diameter of the left two passages is smaller than the four passages on the right side). In other embodiments, the plurality of passages 824 can have about the same diameter.

In some embodiments, when the balloon cover 800 encloses the distal end portion of the balloon catheter 870 (including the balloon 880 mounted thereto), the balloon cover 800 can have a small cavity (e.g., at about the location where the arrow 808 points to in FIG. 22A) configured to receive a distal end of the outer shaft. In some embodiments, this cavity (the proximal portion of shaft section 808 of the cover) is sized to receive a distal portion of an outer shaft and a tip portion of the outer shaft, such as outer shaft 972 and tip portion 973 of FIG. 24. When the balloon cover 800 is in the open state with the distal end portion of the balloon catheter disposed in one of shell members 802a, 802b, slightly pushing the balloon 800 in a proximal direction causes the proximal portion 882 of the folded balloon 800 to "puff up" or more closely conform to the shape of the proximal compartment 814 of the cover, which can help maintain the desired shape of the balloon during storage of the balloon catheter.

According to one exemplary embodiment, the first shell member 802a includes a plurality of first wedges 842a juxtaposed with a plurality of first cavities 844a and the second shell member 802b includes a plurality of second wedges 842b juxtaposed with a plurality of second cavities 844b. The first wedges 842a and first cavities 844a are configured to interlock with the corresponding second cavities 842b and second wedges 844b when the balloon cover 800 is in the closed state.

For example, the first cavities 844a can be so positioned and sized to receive the corresponding second wedges 842b and the second cavities 844b can be so positioned and sized to receive the corresponding first wedges 842a. Thus, when the balloon 880 is in the closed state (see e.g., FIGS. 22B and 23B), the first wedges 842a are offset with the second wedges 842b such that the plurality of first wedges 842a are received by the plurality of second cavities 844b and the plurality of second wedges 842b are received by the plurality of first cavities 844a.

As described herein, the interlocking between the wedges 842a, 842b and corresponding cavities 844a, 844b advantageously allow the balloon cover 800 to hold certain areas of the balloon 880 (e.g., areas contacting with wedges) tighter than others (e.g., areas not contacting with edges), according to design need. In addition, the interlocking nature of the design can mitigate the risk of sliding and/or misaligning between the shell members 802a, 802b (thus reducing the risk of damage to the balloon) during manufacture, transportation, and/or storage of the balloon assembly comprising the balloon 880 and the balloon cover 800.

As shown, the first and second wedges 842a, 842b, and the first and second cavities 844a, 844b are located in the balloon section 810 of the lumen. In the depicted embodiment, the wedges 842a, 842b and cavities 844a, 844b are distributed in the proximal shoulder portion 814, the distal shoulder portion 816, as well as the intermediate portion 818. In other embodiments, the wedges 842a, 842b and cavities 844a, 844b can be distributed in only selected part(s) of the balloon section 810.

As shown, the first wedges 842a and first cavities 844a can be located adjacent and along with the first longitudinal edges 840a, and the second wedges 842b and second cavities 844b can be located adjacent and along with the second longitudinal edges 844b.

As shown, the first wedges 842a can protrude above the first longitudinal edges 840a and the cavities 844a can be recessed below the first longitudinal edges 840a. Similarly, the second wedges 842b can protrude above the second longitudinal edges 840b and the cavities 844b can be recessed below the second longitudinal edges 840b.

In the depicted embodiment, the first wedges 842a and first cavities 844a located along the two opposing first longitudinal edges 840a are symmetric about an axial axis of the first shell member 802a, and the second wedges 842b and second cavities 844b located along the two opposing second longitudinal edges 840b are symmetric about an axial axis of the second shell member 802b. In other embodiments, the wedges 842a, 842b and cavities 844a, 844b may be located asymmetric about respective axial axis of the shell members.

In the depicted embodiment, the wedges 842a, 842b and cavities 844a, 844b are distributed about uniformly along the axial axis of the balloon cover (i.e., the wedges and cavities on a cover member are axially spaced apart from each other at about equal distances). In other embodiments, the wedges 842a, 842b and cavities 844a, 844b can be distributed non-uniformly along the axial axis of the balloon cover. For example, the wedges 842a, 842b and cavities 844a, 844b located in the intermediate portion 818 can be configured to have a density (i.e., smaller distances between adjacent wedges or cavities) than the wedges 842a, 842b and cavities 844a, 844b located on the proximal shoulder portion 814 and/or distal shoulder section 816, or vice versa.

In the depicted embodiment, the wedges 842a, 842b are about the same size and shape. In other embodiments, the wedges 842a, 842b can be configured to have different sizes and/or shapes (and the cavities 844a, 844b can have corresponding sizes and/or shapes to receive the respective wedges 842a, 842b). For example, the cross section of the wedges 842a and/or 842b can be square, rectangle, circle, oval, or other shapes. In another example, the cross section of the wedges 842a and/or 842b in the intermediate portion 818 can be configured to have a smaller (or larger surface area) than the wedges 842a, 842b and cavities 844a, 844b located on the proximal shoulder portion 814 and/or distal shoulder section 816.

In some embodiments, the shell members 802a, 802b can be made of a rigid material (similar to the outer shells 602 and 702 described above) so that the lumen 806 cannot be deformed by partial inflation of the balloon 880. In other embodiments, the shell member 802a, 802b can be made of an elastic material (similar to the inner shells 604 and 704 described above) such that the lumen 806 may partially deform (e.g., increase in diameter) due to partial inflation of the balloon 880. In some embodiments, the shell member 802a, 802b can be configured to function in a similar manner as the inner shells 604 and 704, e.g., by including components similar to the legs 634 or rib portions 734 described above, and then including another outer shell outside the shell member 802a, 802b.

FIGS. 24-25 show the distal end portion of a delivery device 970 for a prosthetic valve, according to another embodiment. The delivery device 970 in the illustrated embodiment comprises an inflatable balloon 980 for expanding a prosthetic heart valve within a patient's body.

As shown, the delivery device 970 includes a first shaft 972 (which is an outer shaft in the illustrated embodiment), a second shaft 976 (which is an intermediate shaft in the illustrated embodiment; also referred to as a "balloon shaft") extending through a lumen of the outer shaft 972, a third shaft 978 (which is an inner shaft in the illustrated embodiment) extending through a lumen of the intermediate shaft 976, and a nosecone 974 connected to the distal end portion of the inner shaft 978. The inner shaft 978 can have a guidewire lumen for receiving a guidewire 979, as known in the art. The balloon 980 can have a proximal end portion 982p affixed to a stepped down end portion of the balloon shaft 976 and a distal end portion 984d affixed to the nosecone 974.

In the depicted embodiment, a flexible tip member 973 is fixedly coupled to a distal end of the outer shaft 972. A distal end of the flexible tip member 973 can have a larger diameter than the diameter of the outer shaft 972. The delivery device 970 can include a shoulder assembly 990 connected to the distal end portion of the inner shaft 978, and at least a distal portion 984 of the balloon 980 can be folded around the shoulder assembly 990.

As shown, the shoulder assembly 990 in the illustrated embodiment includes only a distal shoulder 994 (there is no proximal shoulder, in contrast to the shoulder assembly 890 described above). While the distal portion 984 of the balloon is folded around the distal shoulder 994, a proximal portion 982 of the balloon can be folded directly around a section 992 of the inner shaft 978 that is located proximal to the distal shoulder 994. A valve retaining portion 986 of the balloon 980 (which is located between the proximal portion 982 and the distal portion 984) can be positioned along a portion of the inner shaft 978 that connects the distal shoulder 994 and the shaft section 992. As shown, when the balloon 980 is in a deflated and folded state, the valve retaining portion 986 can have a generally cylindrical shape. As shown, both the proximal end portion 984p of the distal portion 984 and the distal end portion 982d of the proximal portion 982 have larger diameters than the valve retaining portion 986 of the balloon 980. In the depicted embodiment, the largest diameter of the folded balloon 980 can be located at the proximal end portion 984p of the distal portion 984.

The proximal end portions of the shafts 972, 976, and 978 can be coupled to a handle of the delivery 970 (e.g., the handle 102). In certain embodiments, the shafts 972, 976, 978 are moveable axially (in distal and proximal directions) and rotationally relative to each other. Further details of the delivery device 970 are disclosed in U.S. Application No. 63/069,567, filed August 24, 2020.

FIGS. 24-25 show that the balloon 980 can assume different shapes during use.

For example, in the embodiment shown in FIG. 24, the proximal portion 982 of the balloon tapers radially inwardly from its distal end portion 982d to its proximal end portion 982p. In contrast, in the embodiment shown in FIG. 25, the proximal portion 982 of the balloon is shaped to form two parts having different shapes: a proximal part 981 and a distal part 983. While the distal part 983 tapers radially inwardly from its distal end to its proximal end, the proximal part 981 has a generally constant diameter along its length. In one example embodiment, the proximal part 981 and the distal part 983 have equal or substantially equal lengths. In other embodiments, the proximal part 981 and the distal part 983 have different lengths.

In addition, in the embodiment shown in FIG. 24, the distal portion 984 of the balloon tapers radially inwardly from its proximal end portion 984p to its distal end portion 984d. In contrast, in the embodiment shown in FIG. 25, the distal portion 984 of the balloon has a generally gourd or hourglass shape characterized by a radial depression in its middle part. Specifically, the distal portion 984 of the balloon tapers radially inwardly from its proximal end portion 984p toward a radially depressed middle portion 984m, and then tapers radially outwardly toward its distal end portion 984d.

The balloon 980 shaped as shown in FIG. 24 can be retained in the balloon cover 200, 400, 600, 700, or 800 described above. The shape of the balloon 980 as shown in FIG. 25 can be formed by the balloon cover 900 as depicted in FIGS. 26-27 and described more fully below.

Prior to introducing the delivery device 970 into a patient, a prosthetic valve can be crimped onto the valve-retaining portion 986 of the balloon 980. As previously described, prior to crimping, the balloon 980 can undergo a cyclic de-airing process whereby the inflation fluid is introduced into the balloon and then withdrawn from the balloon. The process of introducing inflation fluid into the balloon and then withdrawing the inflation fluid can be repeated one or more times as needed. During the de-airing process, the tip 973 of the outer shaft 972 typically is positioned proximal to the balloon to facilitate the flow of inflation fluid into the proximal portion 982 of the balloon 980. In some embodiments, the de-airing process can be carried out while the balloon 980 is contained within a balloon cover. Following the de-airing process, the balloon cover can be removed from the balloon and the outer shaft 972 can be moved relative to the intermediate shaft 976 (and the inner shaft 978) to a distal position extending over the proximal part 981 of the proximal portion 982 of the balloon (as shown in phantom in FIG. 25). When the outer shaft 972 is moved distally over the proximal part 981, residual fluid in the proximal portion of the balloon from the de-airing process can be pushed distally into the valve-retaining portion 986 and the distal portion 984 of the balloon. Following the de-airing process, the shape of the folded balloon depicted in FIG. 25 can be advantageous than the one depicted in FIG. 24 in at least two aspects.

First, the tapered shape of the proximal portion 982 of balloon shown in FIG. 24 can press against the flexible tip member 973 and resist the distal advancement of the outer shaft 972 relative to the intermediate shaft 976 and the balloon 980 due to the gradual increase of the diameter of the balloon from the proximal end portion 982p to the distal end portion 982d and/or the presence of residual fluid in the proximal portion of the balloon. In contrast, the smaller diameter of cylindrically-shaped proximal part 981 depicted in FIG. 25 facilitates axial movement between the outer shaft 972 and the intermediate shaft 976 with less resistance, especially when the inner diameters of the lumen of the outer shaft 972 and the lumen of the flexible tip member 973 are configured to be about the same as or slightly larger than the outer diameter of the proximal part 981 of the balloon.

Second, when the residual inflation fluid in the proximal portion 980 of the balloon is "squeezed" or pushed into the distal portion 984 of the balloon by advancing the outer shaft 972, the displaced residual fluid can dilate the distal portion 984 of the balloon and increase the outer diameter at the proximal end portion 984p, thus increasing the overall crimp profile of the delivery device 970. Moreover, the effect of the displaced inflation fluids can be even more pronounced when the proximal portion 982 has the tapered shape shown in FIG. 24. In contrast, undesirable inflation of the proximal end portion 984p can be avoided by creating the depression along the middle portion 984m as shown in FIG. 25. Thus, when the displaced inflation fluid is pushed into the distal portion 984 of the balloon, the middle portion 984m can receive the inflation fluid and partially inflate from the hourglass shape (shown in solid lines) to the tapered conical shape 984m' (shown in dashed lines) having a diameter that gradually and continuously decreases from the proximal end portion 984p to the distal end portion 984d (shown in dashed lines Thus, the radially depressed middle portion 984m effectively creates a negative radial depression that can compensate for the displaced residual fluid, thereby preventing or minimizing inflation of the proximal end portion 984p of the balloon distal portion. In this manner, increasing the overall crimp profile of the delivery device 970 as a result of advancing the outer shaft can be avoided.

Generally, the maximum diameter of the balloon and the crimped prosthetic valve typically is the location where the inflow end of the prosthetic valve comes in contact with the proximal end portion 984p of the balloon distal portion and therefore the overall crimp profile of the delivery device can be determined by the diameter at this location. For the balloon shaped as shown in FIG. 24, in some examples, the displaced inflation fluid can increase the diameter of the balloon at the inflow end of the prosthetic valve (at location 984p) by about 0.25 mm. In contrast, for the balloon shaped as shown in FIG. 25, in some examples, the displaced inflation fluid causes a slight increase of the diameter of the balloon at the inflow end of the prosthetic valve (e.g., an increase of about 0.12 mm). Thus, by changing the balloon shape from FIG. 24 to FIG. 25, the increase in diameter of the balloon at the inflow end of the prosthetic valve due to the displaced inflation fluid can be reduced by over 50% (e.g., from about 0.25 mm to about 0.12 mm).

Various techniques and mechanisms can be used to achieve the balloon shape shown in FIG. 25, including a balloon cover having an internal cavity that is shaped to produce the desired shape of the balloon.

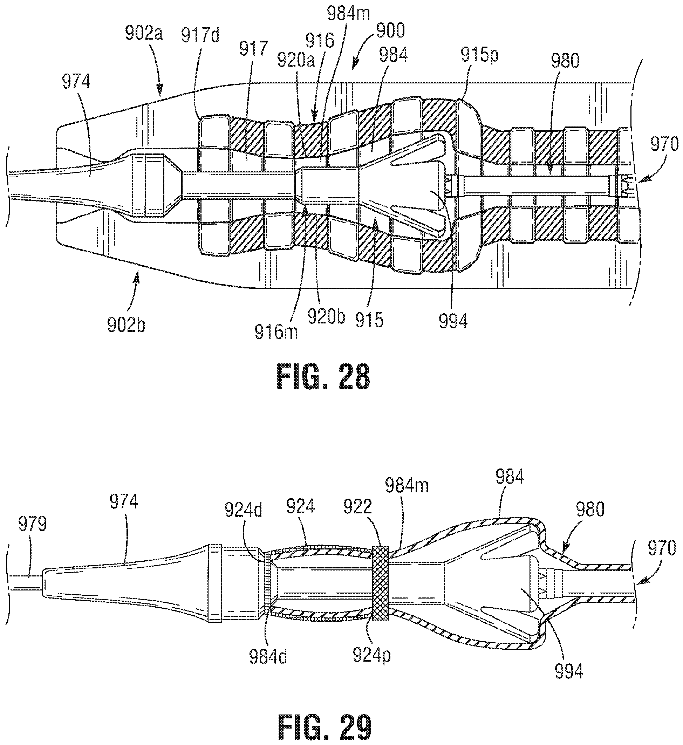

FIGS. 26-27 show a balloon cover 900 configured to receive the delivery device 970 and the balloon 980 folded thereon, according to one embodiment. Specifically, the balloon cover 900 is configured to receive and create the final shape the balloon 980 shown in FIG. 25. For example, FIG. 28 shows a distal portion of the balloon cover 900 that retains the distal portion 984 of the hourglass-shaped balloon depicted in FIG. 25. As described more fully below, the balloon cover 900 is similar to the balloon cover 800 described above, except for the shape of the lumen that is configured to receive the balloon.

As shown, the balloon cover 900 includes a first shell member 902a and a second shell member 902b that are configured to matingly engage each other. When the first and second shell members 902a, 902b are mating engaged with each other, the balloon cover 900 is in a closed state, as shown in FIG. 27. The respective inner surfaces (or "inner walls") 904a, 904b of the shell members 902a, 902b can define a lumen 906 that is configured to receive the distal end portion of the delivery device 970, including the balloon 980 folded thereon. When the shell members 902a, 902b are disengaged from each other, the balloon cover 900 is in an open state, as shown in FIG. 26. The first and second shell members 902a, 902b can be matingly coupled together or separated from each other along respective longitudinal edges 940a, 940b. Similar to the balloon cover 800, the shell members 902a, 902b can be made of a rigid material or an elastic material, and they can be completely separable from or connected to each other with a hinge (e.g., such as shown in FIGS. 13 or 17).

Similar to the balloon cover 800, the first shell member 902a has a plurality of first wedges 942a alternating with a plurality of first cavities 944a along the length of the first shell member. The second shell member 902b has a plurality of second wedges 942b alternating with a plurality of second cavities 944b along the length of the second shell member. The first wedges 942a and first cavities 944a are configured to interlock with corresponding second cavities 944b and second wedges 942b when the balloon cover 900 is in the closed state. In the depicted embodiment, the first wedges 942a and first cavities 944a are perpendicular to the first longitudinal edges 940a, and the second wedges 942b and second cavities 944b are perpendicular to the second longitudinal edges 940b. As shown, the first and second wedges 942a, 942b can protrude above the respective first and second longitudinal edges 940a, 940b, and the first and second cavities 944a, 944b can be recessed below the respective first and second longitudinal edges 940a, 940b when the cover is in the closed state.

Like the balloon cover 800, the lumen 906 of the balloon cover 900 includes a balloon section 910 configured to receive the balloon 980 folded on the distal end portion of the delivery device 970, a shaft section 908 configured to receive a distal end portion of the intermediate shaft 976, and a nosecone section 912 configured to receive the nosecone 974. The balloon section 910 is located between the shaft section 908 and the nosecone section 912. As shown, the balloon section 910 includes a proximal compartment 914 configured to receive the proximal portion 982 of the balloon, a distal compartment 916 configured to receive the distal portion 984 of the balloon, and an intermediate compartment 918 between the proximal and distal compartments 914, 916 and which is configured to receive the valve retaining portion 986 of the balloon. A distal end 914d of the proximal compartment 914 can have a smaller diameter than a proximal end 916p of the distal compartment 916, and the intermediate compartment 918 can have a smaller diameter than the distal end 914d of the proximal compartment 914.

The shaft section 908 and nosecone section 912 of the balloon cover 900 can be similar to the respective shaft section 808 and nosecone section 812 of the balloon cover 800. The intermediate compartment 918 of the balloon cover 900 can be similar to the intermediate portion 818 of the balloon cover 800. However, the proximal compartment 914 and/or the distal compartment 916 of the balloon cover 900 can have different shapes than the corresponding compartments 814, 816 of the balloon cover 800.

Specifically, the proximal compartment 914 can include a proximal part 913 and a distal part 911 that are shaped to receive the corresponding proximal part 981 and distal part 983 of the balloon depicted in FIG. 25. As shown in FIG. 26, the distal part 911 tapers radially inwardly from its distal end to its proximal end, and the proximal part 913 has a substantially constant diameter along its axial length. In the depicted embodiment, the proximal part 913 has about the same axial length as the distal part 911. In other embodiments, the proximal part 913 and the distal part 911 can have different lengths so long as they match the respective lengths of the proximal part 981 and distal part 983 of the balloon.

In addition, as shown in FIG. 26, the distal compartment 916 includes a proximal region 915, a distal region 917, and a middle region 916m between the proximal and distal regions 915, 917. Generally, the distal region 917 has a smaller diameter than the proximal region 915, and the middle region 916m has a smaller diameter than the distal region 917. Specifically, in the illustrated embodiment, the distal compartment 916 tapers radially inwardly from the proximal region 915 to the middle region 916m and then tapers radially outwardly from the middle region 916m to the distal region 917. A distal end 917d of the distal region 917 can have a smaller diameter than a proximal end 915p of the proximal region 915. As best shown in FIG. 28, the distal compartment 916 has a generally gourd or hourglass shape that is configured to receive the distal portion 984 of the balloon depicted in FIG. 25.

In certain embodiments, the deflated balloon can be initially folded to achieve the shape depicted in FIG. 24. The folded balloon can include axial and/or radial folds as shown in FIGS. 6, 8 and 10. A compression mechanism or compression member can be applied to radially compress the distal portion 984 of the balloon to form the hourglass shape depicted in FIG. 25.

In certain embodiments, a radial protrusion from the inner walls of the shell members at the middle region 916m can be used as a compression mechanism to radially compress and retain the middle portion 984m of the balloon distal portion. For example, as shown in FIGS. 26 and 28, a first semicircular radial protrusion 920a can extend radially inwardly from the inner wall 904a of the first shell member 902a and a second semicircular radial protrusion 920b can extend radially inwardly from the inner wall 904b of the second shell member 902b. The first and second semicircular radial protrusions 920a, 920b can form a circular radial protrusion that extends radially inwardly when the first and second shell members 902a, 902b are matingly engaged with each other.

In certain embodiments, the shape of the lumen 906 can be defined by inner surfaces of the wedges 942a, 942b. When the two shell portions 902a, 902b are in the closed state, the distal compartment 916 of the lumen can have a smooth hourglass cross-sectional profile, and the distal portion 984 of the balloon will assume that shape when shell portions 902a, 902b are closed around the balloon. In the embodiment shown in FIG. 28, the radial protrusions 920a, 920b can be defined by inner surfaces of the wedges 942a, 942b, which correspond to the smallest diameter along the middle portion 916m of the distal compartment. Thus, wedges 942a, 942b at the middle portion 916m can function as compression members to compress the distal portion 984 of the balloon to form the hourglass shape depicted in FIG. 25.

In certain embodiments, the balloon cover 900 can further shape the proximal portion 982 of the balloon from the initial shape of FIG. 24 to the final shape of FIG. 25. For example, the proximal portion 982 of the balloon having an initially tapered shape of FIG. 24 can be reformed by the proximal compartment 914 of the balloon cover to the shape shown in FIG. 25 (i.e., having the tapered distal part 983 and the cylindrical proximal part 981) when shell portions 902a, 902b are closed around the balloon.

FIG. 30 illustrates another compression mechanism or compression member configured to radially compress and retain the distal portion 984 of the balloon. As shown, the compression member can include an annular band or ring 922 configured to be placed around the middle portion 984m of the distal portion 984 of the balloon. In one embodiment, the band 922 can be made from a relatively rigid and non-elastomeric material (e.g., a rigid plastic) having a fixed inner diameter equal to the final, desired diameter of the middle portion 984m of the balloon distal portion. In use, the band 922 can be slid over the nosecone 974 and onto the middle portion 984m of the balloon distal portion. When placed on the balloon, the band 922 creates a radial depression along the middle portion 984m. In another embodiment, the band 922 can made from an elastomeric material (e.g., rubber or a synthetic elastomer, such as polyurethane), such as an O-ring, which can expand to facilitate sliding the band over the nosecone. The inner diameter of the band is sized such that when placed on the balloon, the band creates a radial depression along the middle portion 984m of the balloon distal portion. Also, when positioned on the balloon, the elastomeric band can allow for at least partial inflation of the balloon distal portion 984 during the de-airing process. After the de-airing process, the band 922 can be removed from the balloon by sliding it distally over the nosecone 974. In some embodiments, the band 922 can have sufficient elasticity to permit full inflation of the distal portion 984 of the balloon or at least sufficient inflation for full deployment of the prosthetic valve and can be left on the balloon during the implantation procedure.

In another embodiment, the band 922 can be formed by wrapping a flexible cord or string, such as a suture, around the middle portion 984m of the balloon distal portion and tying the two ends of the cord together. The cord can be sufficiently tightened around the middle portion 984m to create the radial depression.

In some embodiments, after the band 922 is placed around the middle portion, the distal end portion of the delivery device 970 can be placed in a balloon cover (including any of the embodiments of balloon covers disclosed herein) for storage and subsequent de-airing.

In certain embodiments, the compression member can include a flexible sheath 924 connected to the band 922, as shown in FIG. 29. The sheath 924 is configured to surround a distal part of the distal portion 984 of the balloon. A distal end 924d of the sheath 924 can be connected to the distal end portion 984d of the distal portion of the balloon and/or the nosecone 974.

In certain embodiments, the sheath 924 can comprise an elastic or deformable material so that it can be radially expanded when the distal portion 984 of the balloon is inflated, such as during the de-airing process. For example, the sheath 924 can be configured to be movable between a radially compressed configuration and a radially expanded configuration. When the sheath 924 is in the radially compressed configuration, the band 922 can radially compress the middle portion 984m of the balloon to create a radial depression. When the sheath 924 is in the radially expanded configuration (e.g., after radially expanding the distal portion 984 of the balloon), the band 922 can be radially expanded. In some embodiments, after the band 922 and the sheath 924 are placed over the balloon, the distal end portion of the delivery device 970 can be placed in a balloon cover (including any of the embodiments of balloon covers disclosed herein) for storage and subsequent de-airing.

After the de-airing process, the band 922 and the sheath 924 can be removed from the balloon by rolling up the band and the sheath in the distal direction until the rolled-up band and sheath is on the nosecone. In some embodiments, the band 922 and the sheath 924 can allow for sufficient inflation of the balloon for valve deployment and can be kept in place over the balloon during the implantation procedure.

In some embodiments, there is no separate structure forming the band 922. Instead, the band 922 can be an integral portion of the proximal end portion 924p of the sheath 924. In one example, the sheath 924 can be relatively thicker and less resilient along the proximal end portion 924p compared to remaining portion of the sheath 924 such that the proximal end portion 924p can apply a sufficient inward force on the middle portion 984m of the balloon distal portion to create a radial depression along that portion of the balloon.

Although in the examples described above, a compression mechanism or compression member is disposed in the distal compartment of the balloon cover to radially compress the distal portion of the balloon, it is to be understood that similar compression mechanisms or compression members can also be placed at other locations (e.g., the proximal compartment and/or the intermediate compartment) of the balloon cover depending on the needs of the design (e.g., to radially compress a certain portion of the balloon to conform to a specified diameter or shape at that portion). For example, it may be desirable to create a depression in the proximal portion of a balloon, which can facilitate sliding of the outer shaft 972 over the proximal portion of the balloon.

In certain embodiments, for example, the proximal compartment 914 and/or the intermediate compartment 918 can be formed with a compression member (e.g., a radial protrusion) to create a depression in the portions of the balloon in those compartments. In some embodiments, the cover 900 can include one or more compression members (e.g., radial protrusions) at one or more locations along the length of the cover, including the proximal compartment 914, the intermediate compartment 918, and/or the distal compartment 916. Similarly, the device of FIG. 29 can be sized such that the band 922 can be placed around any portion of the balloon, including the proximal portion, the intermediate portion or the distal portion of the balloon.

It should be understood that the compression mechanisms described above are merely example embodiments. Other embodiments can be employed to radially compress the middle portion 984m of the balloon or other portions of the balloon so that the compressed section of the balloon has a generally gourd or hourglass shape.

As described below, the balloon cover 600 (or cover 200, 400, 700, 800, or 900) can facilitate de-airing of the balloon 680 by employing a compressible inner member or layer defining the inner chamber for receiving a balloon.

Prior to crimping a prosthetic valve onto the balloon, the balloon typically needs to undergo a cyclic de-airing process that involves pushing an inflation fluid into the balloon and then drawing the fluid out (e.g., with a syringe) to create a vacuum in the balloon. The de-airing process is more successful when the balloon is allowed to at least partially inflate. However, inflation of a folded balloon without a balloon cover can result in un-folding of the balloon, which may not then return to its compressed, folded shape. On the other hand, rigid balloon covers may prevent inflation of the balloon entirely.

The balloon cover 600 (or 200, 400 700, 800, or 900) disclosed herein can overcome such problems. Advantageously, the flexible inner sleeve 604 (or 704; similarly, covers 200, 400, 700 800, 900 can include a flexible or deformable inner sleeve or layer forming the chamber for receiving the balloon) allows the folded balloon 680 to partially inflate within the inner sleeve 604 (or 704, or similar components of covers 200, 400, 700, 800, 900) during the de-airing process without becoming unfolded. Specifically, when an inflation fluid is injected into the folded balloon 680, the balloon 680 can partially inflate, pressing against the inner sleeve 604 which deforms (e.g., by increasing the diameter of the lumen and shortening the legs) to accommodate the partially inflated balloon 680. The degree of flexibility of the inner sleeve 604 limits the partial inflation of the balloon 680. For example, the maximum diameter the balloon 680 can be inflated to can be limited by the largest diameter of the lumen 608 (or rib enclosures 750, or 810) can be extended to, which can be further limited by the shortest radial length the legs 634 can be compressed to and is inherently limited by the diameter of the intermediate recess 624 of the outer shell 602.

When the vacuum is created in the balloon 680 after drawing the inflation fluid out of the balloon, the flexible inner sleeve 604 (or 704, or similar components of covers 200, 400, 700, 800, 900) can revert back to its original shape (e.g., by decreasing the diameter of the lumen 608 (or rib enclosures 750, or 810) and lengthening the legs 634). Thus, the contracting inner sleeve 604 (or 704, 800, 900) can press inwardly against the balloon 680 and help return the balloon 680 to its original (folded) shape.

An example method of assembling the balloon 680 within the balloon cover 600 is described below (and similar method can also be applied to assemble the balloon 680 within the balloon cover 200, 400, 700, 800, or 900).

According to one embodiment, the balloon 680 (in the deflated state) can be folded over the shoulder assembly (including the proximal shoulder 690, the distal shoulder 692 and the valve retaining portion 686) of the delivery device 670 as shown in FIG. 19. The folded balloon 680 can be placed within the lumen 608 of the inner sleeve 604, e.g., by aligning the corresponding longitudinal edges 618a, 618b of the first and second portions 604a, 604b to enclose the valve retaining portion 686 of the balloon 680. Then, the inner sleeve 604 and the balloon 680 can be placed within the outer shell 602. For example, the shell members 602a, 602b can be placed around the balloon 680 such that the proximal end portion 682 of the balloon 680 is placed inside the proximal recess 620 of the outer shell 602, the distal end portion 684 of the balloon is placed inside the distal recess 622 of the outer shell 602, and the inner sleeve 604 is placed inside the intermediate recess 624 of the outer shell 602. Then, the outer shell 602 can be locked, e.g., by extending the enlarged rim 614a through the slot 614b to form a snap-fit between the shell members 602a, 602b.

An example method of de-airing of the balloon 680 inside the balloon cover 600 is described below, and it should be understood that the same method can also apply to de-airing the balloon inside the other balloon covers disclosed herein.

According to one embodiment, the balloon 680 can be fluidly connected to a fluid source (e.g., a syringe). The fluid source can inject a predetermined amount of inflation fluid (e.g., saline) into the balloon 680 so as to inflate the balloon 680 to a partially inflated state. In the partially inflated state, the balloon 680 can still remain partially folded (e.g., at least some of the adjacent folds remain partially overlapped with each other) and can revert back to its original (folded) shape after removal of the inflation pressure. As described above, inflation of the balloon 680 can deform the lumen 608 of the inner sleeve 604 from its initial state (with a relatively smaller diameter) to the deformed state (with a relatively larger diameter). Then, the inflation fluid can be removed from the balloon 680 (e.g., withdrawn back into the inflation source) to create a vacuum inside the balloon 680. The vacuum can cause the balloon 680 to return from its partially inflated state to the deflated state. The lumen 608 of the inner sleeve 604 can also revert back from the deformed state (with a relatively larger diameter) to its initial state (with a relatively smaller diameter), thereby press inwardly against the balloon 680 and help return the balloon 680 to its original (folded) shape. In some embodiments, the steps of (a) inflating the balloon 680 from the deflated state to the partially inflated state and (b) deflating the balloon 680 from the partially inflated state to the deflated state can be repeated in a plurality of cycles.

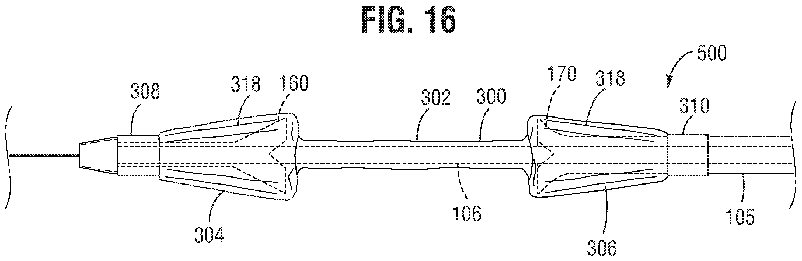

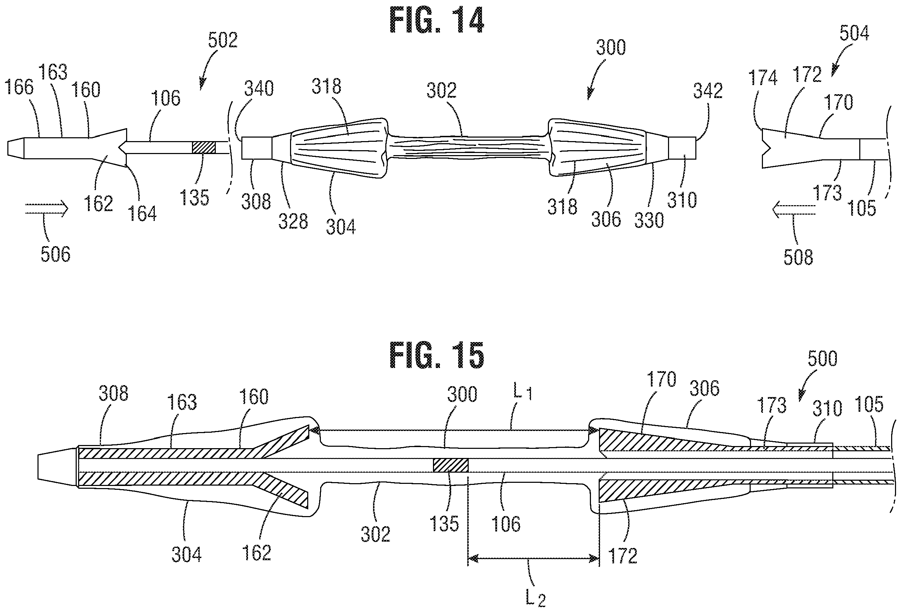

FIG. 14 illustrates a method of assembling a delivery device to include the balloon 300, according to one embodiment. FIGS. 15-16 show a fully assembled delivery device 500 with the folded balloon 300 mounted thereon. The delivery device 500 includes many of the same components of the delivery device 100 of FIGS. 3-7. Thus, the same components in FIGS. 15-16 are given the same respective reference numbers and are not described further.

Although the balloon 300 is shown in FIGS. 14-16 for purposes of illustration, it should be understood that the method described below can be to assemble a delivery device having other balloons, such as the balloon 108, or a balloon that does not have any folds. Although less desirable, in other embodiments, a balloon can be folded after being assembled onto a delivery device. For example, the balloon 300 without any folds (e.g., folds 316, 318, 322, 326) can be assembled onto a delivery device (as described below), after which the balloon can be folded.

Referring to FIG. 14, a distal shoulder 160 (which can include a nosecone portion 166) can be mounted on the distal end portion of a first shaft 106 (which can be the inner shaft of the delivery device) to form a first sub-assembly 502 of the delivery device. A proximal shoulder 170 can be mounted on the distal end portion of a second shaft 105 (which can be an intermediate shaft of the delivery device) to form a second sub-assembly 504 of the delivery device.

FIG. 14 shows the balloon 300 removed from its protective cover 400 during the assembly process. In other embodiments, the protective cover 400 (or cover 200, 600, 700, 800, or 900) can be kept in place surrounding the balloon during the assembly process. The restraining member 350 (not shown in FIG. 14) can be removed from the balloon just prior to the assembly process described below or after the balloon is assembled onto the delivery device, or alternatively, the restraining member 350 can be left in place for removal by the end user prior to crimping a prosthetic valve onto the balloon.

In the direction indicated by arrow 506 in FIG. 14 (left to right in FIG. 14), the proximal end of the first shaft 106 can be inserted into an open distal end 340 of the distal leg 308. The first shaft can then be inserted through the balloon 300, through the proximal shoulder 170, and through the second shaft 105, after which the distal shoulder 160 can be at least partially inserted into the distal tapered portion 304 of the balloon. Because the distal collar member 162 is larger in diameter than the distal leg 306, the distal collar member 162 can be radially compressed, and then inserted into and through the distal collar member 162. The distal shoulder 160 can be inserted into the balloon until the distal leg 308 extends over the shaft portion 163 of the distal shoulder 160. Because the distal collar member 162 is made of a resilient material, it can radially expand back to its original shape once it is passed through the distal leg 308. The distal leg 308 of the balloon can then be secured to the distal shoulder 160 (e.g., to the shaft portion 163) by means of thermal bonding, gluing, mechanical locking, or any other suitable techniques or methods.

In the direction indicated by arrow 508 in FIG. 14 (right to left in FIG. 14), the proximal shoulder 170 can be inserted into an open distal end 342 of the proximal leg 310 of the balloon and inserted through the balloon until the proximal collar member 172 resides within the proximal tapered portion 306 of the balloon and the proximal leg 310 extends over the shaft portion 173 of the proximal shoulder 170. Because the proximal collar member 172 is larger in diameter than the proximal leg 310, the proximal collar member 172 can be radially compressed before it is inserted into and through the proximal leg 310. Because the proximal collar member 172 is made of a resilient material, it can radially expand back to its original shape once it is passed through the proximal leg 310. The proximal leg 310 of the balloon can then be secured to the proximal shoulder 170 (e.g., to the shaft portion 173) by means of thermal bonding, gluing, mechanical locking, or any other suitable techniques or methods.

For purposes of illustration, there are shown small gaps between the legs 308, 310 and the shaft portions 163, 173. However, it should be understood, the legs 308, 310 can be in contact with the shaft portions 163, 73 once they are secured to the shaft portions 163, 173.

One or more components of the balloon 300 and the distal and proximal shoulders 160, 170 can include markings or indicia to facilitate alignment of the balloon with respect to the distal and proximal shoulders 160, 170. For example, the distal leg 308 can include one or more markings that are aligned with corresponding one or more markings on the shaft portion 163 of the distal shoulder 160. Likewise, the proximal leg 308 can include one or more markings that are aligned with corresponding one or more markings on the shaft portion 173 of the proximal shoulder 170. Once the distal and proximal legs 308, 310 are aligned with the shaft portions 163, 173, respectively, using the markings, the mating components can be affixed to each other as described above. The markings can be any type of marking, including markings printed or painted on the components, markings formed by laser etching, or markings molded into the components.

The required axial distance L1 between the distal shoulder 160 and the proximal shoulder 170, and therefore the distance between the distal tapered portion 304 and the proximal tapered portion 306 of the balloon, can be predetermined and can be selected to allow a prosthetic valve to be crimped between the distal tapered portion 304 and the proximal tapered portion 306. Desirably, the axial distance L1 is selected such that when the prosthetic valve is crimped onto the balloon, the distal tapered portion 304 and the proximal tapered portion 306 can contact adjacent ends of the crimped prosthetic valve. When assembling the first sub-assembly 502 with the second sub-assembly 504, this distance can be set by measuring a predetermined offset between a component on the first sub-assembly 502 with a component on the second sub-assembly 504, such as a distance L2 between the proximal shoulder 170 and the marker band 135. For example, when inserting the shaft 106 into the shaft 105, the position of the shaft 106 relative to the shaft 105 can be adjusted until the distance between the proximal shoulder 170 and the marker band 135 is a predetermined distance L2, which corresponds to a predetermined distance L1 between the distal and proximal tapered portions 304, 306 of the balloon.

In alternative embodiments, other offsets can be used to set the distance L1, including (but not limited to): (i) an axial distance between the proximal shoulder 170 and the distal shoulder 160; (ii) an axial distance between the proximal shoulder 170 and the distal end of the valve retaining portion 302 of the balloon; and (iii) an axial distance between the distal shoulder and the proximal end of the valve retaining portion 302 of the balloon.

In certain embodiments, the axial position of the shaft 106 relative to the shaft 105 is fixed, such as by securing these components directly to each other, such as by welding, an adhesive, mechanical fasteners, etc. The proximal end portions of the shafts 105, 106 can then be coupled to a handle (e.g., handle 102). Alternatively, the proximal end portions of the shafts 105, 106 can be secured to a handle (e.g., handle 102) at fixed locations, which fixes the positions of the shafts 105, 106 relative to each other.

In some embodiments, a steerable outer shaft 104 (such as shown in FIG. 3), can be assembled over the shafts 105, 106.

As noted above, in some embodiments, the protective cover 400 (or cover 200, 600, 700, 800, or 900) can be kept in place surrounding the balloon 300 during the assembly process. As shown in FIG. 13, the cover 400 has distal and proximal openings 414, 416, which can be sized to allow components of the delivery device to be inserted into the cover 400 and through the openings 340, 342 of the balloon while the balloon is inside of the cover 400.

The cover 400 can facilitate alignment of the balloon with components of the delivery apparatus. For example, it sometimes can be difficult for the assembler to visually locate specific locations on the balloon 300 when aligning the balloon relative to the distal and proximal shoulders 160, 170. Instead, the assembler can visually locate specific portions on the cover and align those portions with the distal and proximal shoulders (or other components of the delivery apparatus to which the balloon is secured).

For example, a specific location on the distal recessed portion 408 of the cover corresponding to a specific location of the leg portion 308 disposed within the recessed portion 408 can be aligned with a predetermined location of the distal shoulder 160. Similarly, a specific location of the proximal recess portion 412 corresponding to a specific location of the leg portion 310 disposed within the recessed portion 412 can be aligned with a predetermined location of the proximal shoulder 170. Optionally, one or more markers can be included on the cover 400 at these locations to further facilitate the alignment process. Moreover, because the cover 400 is more rigid than the balloon 300, the cover 400 can further facilitate the alignment process because the balloon can be more easily manipulated (e.g., pushed and pulled) relative to the sub-assemblies 502, 504 when positioning and aligning the balloon by grasping and moving the cover instead of grasping the balloon directly.

Once the balloon 300 is aligned relative to the predetermined locations on the distal and proximal shoulders (or other locations on the sub-assemblies 502, 504), the assembler optionally can open the cover 400 prior to affixing the distal and proximal legs 308, 310 to the distal and proximal shoulders 160, 170, such as by welding or applying an adhesive to these components. After securing the balloon in place, the cover 400 can be re-closed and left in place for the remainder of the assembly process and/or for shipping and storage.

If the cover is removed from the balloon prior to assembly, the cover 400 can be placed back on the balloon 300 for shipping and storage. Alternatively, another cover can be placed around the balloon if the initial cover 400 is not re-useable. The assembly of the delivery device and the cover can be further placed in a sterile package for shipping and storage, as known in the art. It should be understood that the features and use of the cover 400 described above can apply to covers 200, 600, 700, 800 and 900.

An off-device formed balloon, such as balloon 300 described above, has a number of advantages. For example, a finished off-device balloon can be inspected to verify that it matches dimensional specifications prior to being assembled onto the delivery device. Importantly, off-device balloons can be produced in large numbers and their performance can be tested on dummy devices prior to being released to stock and used to manufacture finished delivery devices. This will not only reduce the variability in balloon performance due to manufacturing parameter shifts, but also allow volume production of the off-device formed balloons in one facility and then transport of the balloons to another facility for mounting of the balloons to delivery devices. The latter can automate the delivery device assembly process and improve the production capacity due to the elimination of time-consuming balloon forming steps on the catheter manufacturing line. Further, the manufacturing line for assembling the delivery device requires less physical space and fewer production operators.

Thus, in certain embodiments, a manufacturer can produce any number of fully formed and folded balloons (e.g., balloons 300), and optionally place the balloons in covers (e.g., covers 200, 400, 600, 700, 800, and 900). The preformed and pre-folded balloons can then be placed into stock for assembling delivery devices in another manufacturing area at the assembly site or shipped to another facility for assembling the delivery devices if the preformed and pre-folded balloons are produced at a different site from where the delivery devices are assembled.

In certain embodiments, a prosthetic valve (e.g., valve 10 or 50) intended for use with a certain delivery device can be stored in a container or jar containing a hydrating fluid. At the point of use, the user can remove the prosthetic valve from its container, remove the delivery device from its packaging, place the prosthetic valve around the balloon (e.g., balloon 108 or 300) and crimp the prosthetic valve onto the balloon between the distal and proximal shoulders (e.g., shoulders 160, 170). In some embodiments, the prosthetic valve can be crimped on the delivery device at a location offset from the valve-mounting portion of the balloon, such as at a location on the proximal portion of the balloon, or on a portion of a shaft proximal to the balloon, such as disclosed in U.S. Publication No. 2013/0030519. Once inserted into the body and prior to deployment, the prosthetic valve can be moved (e.g., pushed) from its crimped location to the valve-mounting portion of the balloon.

In alternative embodiments, a prosthetic valve can have dry or substantially dry leaflets that can be stored without a hydrating fluid, such as disclosed in U.S. Patent Nos. 8,007,992 and 8,357,387. In such cases, the prosthetic valve can be crimped onto the balloon of a delivery device (or at a location offset from the balloon) by the manufacturer and the assembly comprising the delivery device and the crimped prosthetic valve can be placed in sterile package for shipping and storage until use by a health care provider. For example, in some cases, a prosthetic valve can be crimped onto a balloon at the same site where the delivery device is assembled, or alternatively, the fully assembled delivery device can be shipped to another facility where the prosthetic valve is crimped onto the delivery device and packaged for delivery to an end user.

Any of the delivery devices disclosed herein can be used to implant a prosthetic valve (e.g., valve 10 or 50) to a native valve (the aortic, mitral, tricuspid, or pulmonary valve) using any of various delivery techniques. For example, when implanting the prosthetic valve within the native aortic valve, the delivery apparatus and the prosthetic valve can be inserted into and through a femoral artery, and through the aorta to the native aortic valve (known as transfemoral delivery). Once the prosthetic valve is positioned at the desired implantation site, the balloon can be inflated to radially expand the prosthetic valve into engagement with surrounding tissue of the native valve. In another delivery approach, the delivery apparatus and the prosthetic valve can be inserted through a surgical opening in the apex of the heart and advanced to position the prosthetic valve within the native aortic valve (known as transapical delivery).

### General Considerations

It should be understood that the disclosed embodiments can be adapted to deliver and implant prosthetic devices in any of the native annuluses of the heart (e.g., the pulmonary, mitral, and tricuspid annuluses), and can be used with any of various delivery approaches (e.g., retrograde, antegrade, transseptal, transventricular, transatrial, etc.).

For purposes of this description, certain aspects, advantages, and novel features of the embodiments of this disclosure are described herein. The disclosed methods, apparatus, and systems should not be construed as being limiting in any way. Instead, the present disclosure is directed toward all novel and nonobvious features and aspects of the various disclosed embodiments, alone and in various combinations and sub-combinations with one another. The methods, apparatus, and systems are not limited to any specific aspect or feature or combination thereof, nor do the disclosed embodiments require that any one or more specific advantages be present or problems be solved. The technologies from any example can be combined with the technologies described in any one or more of the other examples. In view of the many possible embodiments to which the principles of the disclosed technology may be applied, it should be recognized that the illustrated embodiments are only preferred examples and should not be taken as limiting the scope of the disclosed technology.

Although the operations of some of the disclosed embodiments are described in a particular, sequential order for convenient presentation, it should be understood that this manner of description encompasses rearrangement, unless a particular ordering is required by specific language set forth below. For example, operations described sequentially may in some cases be rearranged or performed concurrently. Moreover, for the sake of simplicity, the attached figures may not show the various ways in which the disclosed methods can be used in conjunction with other methods. Additionally, the description sometimes uses terms like "provide" or "achieve" to describe the disclosed methods. These terms are high-level abstractions of the actual operations that are performed. The actual operations that correspond to these terms may vary depending on the particular implementation and are readily discernible by one of ordinary skill in the art.

As used herein, with reference to the prosthetic valve, delivery apparatus and other components of the delivery assembly, "proximal" refers to a position, direction, or portion of a device that is closer to the handle of the delivery assembly that is outside the patient, while "distal" refers to a position, direction, or portion of a device that is further away from the handle. The terms "longitudinal" and "axial" refer to an axis extending in the proximal and distal directions, unless otherwise expressly defined.

As used in this application and in the claims, the singular forms "a," "an," and "the" include the plural forms unless the context clearly dictates otherwise. Additionally, the term "includes" means "comprises." Further, the terms "coupled" and "connected" generally mean electrically, electromagnetically, and/or physically (e.g., mechanically or chemically) coupled or linked and does not exclude the presence of intermediate elements between the coupled or associated items absent specific contrary language.

Directions and other relative references (e.g., inner, outer, upper, lower, etc.) may be used to facilitate discussion of the drawings and principles herein, but are not intended to be limiting. For example, certain terms may be used such as "inside," "outside,", "top," "down," "interior," "exterior," and the like. Such terms are used, where applicable, to provide some clarity of description when dealing with relative relationships, particularly with respect to the illustrated embodiments. Such terms are not, however, intended to imply absolute relationships, positions, and/or orientations. For example, with respect to an object, an "upper" part can become a "lower" part simply by turning the object over. Nevertheless, it is still the same part and the object remains the same. As used herein, "and/or" means "and" or "or", as well as "and" and "or".

## Claims

1. A balloon assembly for a transcatheter delivery device configured for implanting a medical device, comprising:
an inflatable balloon (108) having an inflated state and a deflated state,
wherein the inflatable balloon (108) in the deflated state comprises:
a valve retaining portion (144) having a generally cylindrical shape and one or more axial folds (146);
a distal tapered portion (142) connected to a distal end of the valve retaining portion (144), a proximal end (150) of the distal tapered portion (142) having a larger diameter (D1) than a distal end (152) of the distal tapered portion (142) and the valve retaining portion (144);
a proximal tapered portion (140) connected to a proximal end of the valve retaining portion, a distal end (154) of the proximal tapered portion (140) having a larger diameter (D2) than a proximal end (156) of the proximal tapered portion (140) and the valve retaining portion (130);
a distal leg (180) connected to the distal end (152) of the distal tapered portion (142); and
a proximal leg (182) connected to the proximal end (156) of the proximal tapered portion (140);
wherein the distal tapered portion (142) comprises one or more axial folds (143), the proximal tapered portion (140) comprises one or more axial folds (145),
wherein the proximal end (150) of the distal tapered portion (142) comprises a first radial fold (184) connected to a second radial fold (185), the first and
second radial folds (184, 185) forming a pocket extending distally at the proximal end (150) of the distal tapered portion (142), and
wherein the distal end (154) of the proximal tapered portion (140) comprises a radial fold (188) that forms an obtuse or right angle relative to the valve retaining portion (144).

2. The balloon assembly of claim 1, further comprising a restraining member (350) extending around an outer surface of the valve retaining portion (144) so as to prevent the one or more axial folds (146) of the valve retaining portion (144) from unfolding.

3. The balloon assembly of any of claims 1 to 2, further comprising a cover (200) that encloses the inflatable balloon (108), wherein the cover (200) comprises a proximal end portion (210), a distal end portion (206), and an intermediate portion (208) between the proximal end portion (210) and the distal end portion (206), wherein the proximal end portion (210) defines a proximal recess that is shaped to substantially match a shape of the proximal tapered portion (140) of the inflatable balloon (108), the distal end portion (206) defines a distal recess that is shaped to substantially match a shape of the distal tapered portion (142) of the inflatable balloon (108), and the intermediate portion (208) defines an intermediate recess that is shaped to substantially match a shape of the valve retaining portion (144) of the inflatable balloon (108).

4. The balloon assembly of claim 3, wherein the cover (200) comprises a first cover piece (202a) and a second cover piece (202b) that are complimentarily structured such that they can be matingly assembled together for enclosing the inflatable balloon (108) in the deflated state.

5. The balloon assembly of claim 4, further comprising an outer sleeve (204) extending over the cover (200) so as to prevent the first and second cover pieces (202a, 202b) from separating from each other.

6. The balloon assembly of any of claims 1 to 5, wherein the inflatable balloon (108) comprises an internal lumen extending through the tapered proximal portion (140), the valve retaining portion (144), and the tapered distal portion (142).

7. The balloon assembly of any of claims 1 to 6, wherein the proximal tapered portion (140) of the inflatable balloon (108) comprises a proximal end portion, wherein the one or more axial folds (145) of the proximal tapered portion (140) do not extend into the proximal end portion of the proximal tapered portion (140).

8. The balloon assembly of any of claims 1 to 7, wherein the distal tapered portion (142) of the inflatable balloon (108) comprises a distal end portion, wherein the one or more axial folds (143) of the distal tapered portion (142) do not extend into the distal end portion of the distal tapered portion (142).

9. A method for forming a balloon (108) for a transcatheter delivery device configured for implanting a medical device, the method comprising:
forming an inflatable balloon (108) in a deflated state, the balloon (108) in the deflated state having a valve retaining portion (144), a distal tapered portion (142) connected to a distal end of the valve retaining portion (144), a proximal tapered portion (140) connected to a proximal end of the valve retaining portion (144), a distal leg (180) connected to a distal end of the distal tapered portion (142), and a proximal leg (182) connected to a proximal end of the proximal tapered portion (140);
inserting a distal shoulder (160) mounted on a first shaft (104) of the delivery device (100) through the distal leg (180) and into the distal tapered portion (142) of the inflatable balloon (108);
inserting a proximal shoulder (120) mounted on a second shaft (105) of the delivery device (100) through the proximal leg (182) and into the proximal tapered portion (140) of the inflatable balloon (108);
securing the distal leg (180) of the inflatable balloon (108) to the distal shoulder (160); and
securing the proximal leg (182) of the inflatable balloon (108) to the second shaft (105) and/or the proximal shoulder (120),
wherein forming the inflatable balloon (108) comprises:
forming the valve retaining portion (144) in a generally cylindrical shape, the diameter of which being smaller than both a diameter of a proximal end of the distal tapered portion (142) and a diameter of a distal end of the proximal tapered portion (140),
forming one or more axially extending folds (143, 145) along at least sections of the distal tapered portion (142) and the proximal tapered portion (140),
forming a radial fold (188) at the distal end of the proximal tapered portion (140), the radial fold (188) forming an obtuse or right angle relative to the valve retaining portion (144),
and
forming a first radial fold (184) connected to a second radial fold (185) at the proximal end (150) of the distal tapered portion (142), the first and second radial folds (184, 185) forming a pocket extending distally at the proximal end (150) of the distal tapered portion (142).

10. The method of claim 9, further comprising placing a restraining member (350) around the valve retaining portion (144).

11. The method of any of claims 9 to 10, further comprising enclosing the inflatable balloon (108) with a cover (200), wherein the cover (200) comprises a first cover piece (202a) and a second cover piece (202b) that are complimentarily structured such that they can be matingly assembled together for enclosing the inflatable balloon (108), and wherein the cover (200) defines an internal recess whose geometry substantially matches a shape of the inflatable balloon (108) in the deflated state.

12. The method of any of claims 9 to 11, wherein a proximal end of the distal shoulder (160) has a larger diameter than a diameter of the distal leg (180), and inserting the distal shoulder (160) through the distal leg (180) comprises radially compressing the proximal end of the distal shoulder (160).

13. The method of any of claims 9 to 12, wherein a distal end of the proximal shoulder (170) has a larger diameter than a diameter of the proximal leg (182), and inserting the proximal shoulder (170) through the proximal leg (182) comprises radially compressing the distal end of the proximal shoulder (170).

14. A prosthetic valve delivery assembly, comprising:
a delivery device (100) comprising:
a shaft (105); and
an inflatable balloon (108) as defined in any of claims 1 to 8 mounted on the shaft (105) in a deflated state.

15. The prosthetic valve delivery assembly of claim 14, further comprising a restraining member (350) extending around an outer surface of the valve retaining portion (144).

## Patentansprüche

1. Ballonanordnung für eine Transkatheter-Zuführvorrichtung, die für die Implantation einer medizinischen Vorrichtung konfiguriert ist, umfassend:
einen aufpumpbaren Ballon (108), der einen aufgepumpten Zustand und einen entleerten Zustand aufweist,
wobei der aufpumpbare Ballon (108) in dem entleerten Zustand umfasst:
einen Klappenhalteabschnitt (144), der eine allgemein zylindrische Form und eine oder mehrere axiale Falten (146) aufweist;
einen distalen verjüngten Abschnitt (142), der mit einem distalen Ende des Klappenhalteabschnitts (144) verbunden ist, wobei ein proximales Ende (150) des distalen verjüngten Abschnitts (142) einen größeren Durchmesser (D1) aufweist als ein distales Ende (152) des distalen verjüngten Abschnitts (142) und des Klappenhalteabschnitts (144);
einen proximalen verjüngten Abschnitt (140), der mit einem proximalen Ende des Klappenhalteabschnitts verbunden ist, wobei ein distales Ende (154) des proximalen verjüngten Abschnitts (140) einen größeren Durchmesser (D2) aufweist als ein proximales Ende (156) des proximalen verjüngten Abschnitts (140) und des Klappenhalteabschnitts (130);
einen distalen Schenkel (180), der mit dem distalen Ende (152) des distalen verjüngten Abschnitts (142) verbunden ist; und
einen proximalen Schenkel (182), der mit dem proximalen Ende (156) des proximalen verjüngten Abschnitts (140) verbunden ist;
wobei der distale verjüngte Abschnitt (142) eine oder mehrere axiale Falten (143) umfasst, und der proximale verjüngte Abschnitt (140) eine oder mehrere axiale Falten (145) umfasst,
wobei das proximale Ende (150) des distalen verjüngten Abschnitts (142) eine erste radiale Falte (184) umfasst, die mit einer zweiten radialen Falte (185) verbunden ist, wobei die erste und die zweite radiale Falte (184, 185) eine Tasche bilden, die sich an dem proximalen Ende (150) des distalen verjüngten Abschnitts (142) nach distal erstreckt, und
wobei das distale Ende (154) des proximalen verjüngten Abschnitts (140) eine radiale Falte (188) umfasst, die einen stumpfen oder rechten Winkel in Bezug auf den Klappenhalteabschnitt (144) bildet.

2. Ballonanordnung nach Anspruch 1, die ferner ein Hemmelement (350) umfasst, das sich um eine Außenfläche des Klappenhalteabschnitts (144) erstreckt, um zu verhindern, dass sich die eine oder mehreren axialen Falten (146) des Klappenhalteabschnitts (144) entfalten.

3. Ballonanordnung nach einem der Ansprüche 1 bis 2, ferner umfassend eine Abdeckung (200), die den aufpumpbaren Ballon (108) umschließt, wobei die Abdeckung (200) einen proximalen Endabschnitt (210), einen distalen Endabschnitt (206) und einen Zwischenabschnitt (208) zwischen dem proximalen Endabschnitt (210) und dem distalen Endabschnitt (206) umfasst, wobei der proximale Endabschnitt (210) eine proximale Aussparung definiert, die so geformt ist, dass sie im Wesentlichen zu einer Form des proximalen verjüngten Abschnitts (140) des aufpumpbaren Ballons (108) passt, wobei der distale Endabschnitt (206) eine distale Aussparung definiert, die so geformt ist, dass sie im Wesentlichen zu einer Form des distalen verjüngten Abschnitts (142) des aufpumpbaren Ballons (108) passt, und wobei der Zwischenabschnitt (208) eine Zwischenaussparung definiert, die so geformt ist, dass sie im Wesentlichen zu einer Form des Klappenhalteabschnitts (144) des aufpumpbaren Ballons (108) passt.

4. Ballonanordnung nach Anspruch 3, wobei die Abdeckung (200) ein erstes Abdeckungsstück (202a) und ein zweites Abdeckungsstück (202b) umfasst, die komplementär strukturiert sind, so dass sie passend zusammengefügt werden können, um den aufpumpbaren Ballon (108) im entleerten Zustand zu umschließen.

5. Ballonanordnung nach Anspruch 4, die ferner eine äußere Hülle (204) umfasst, die sich über die Abdeckung (200) erstreckt, um zu verhindern, dass sich das erste und das zweite Abdeckungsstück (202a, 202b) voneinander trennen.

6. Ballonanordnung nach einem der Ansprüche 1 bis 5, wobei der aufpumpbare Ballon (108) ein inneres Lumen umfasst, das sich durch den verjüngten proximalen Abschnitt (140), den Klappenhalteabschnitt (144) und den verjüngten distalen Abschnitt (142) erstreckt.

7. Ballonanordnung nach einem der Ansprüche 1 bis 6, wobei der proximale verjüngte Abschnitt (140) des aufpumpbaren Ballons (108) einen proximalen Endabschnitt umfasst, wobei sich die eine oder mehreren axialen Falten (145) des proximalen verjüngten Abschnitts (140) nicht in den proximalen Endabschnitt des proximalen verjüngten Abschnitts (140) erstrecken.

8. Ballonanordnung nach einem der Ansprüche 1 bis 7, wobei der distale verjüngte Abschnitt (142) des aufpumpbaren Ballons (108) einen distalen Endabschnitt umfasst, wobei sich die eine oder mehreren axialen Falten (143) des distalen verjüngten Abschnitts (142) nicht in den distalen Endabschnitt des distalen verjüngten Abschnitts (142) erstrecken.

9. Verfahren zum Bilden eines Ballons (108) für eine Transkatheter-Zuführvorrichtung, die zum Implantieren einer medizinischen Vorrichtung konfiguriert ist, wobei das Verfahren umfasst:
Bilden eines aufpumpbaren Ballons (108) in einem entleerten Zustand, wobei der Ballon (108) in dem entleerten Zustand einen Klappenhalteabschnitt (144), einen distalen verjüngten Abschnitt (142), der mit einem distalen Ende des Klappenhalteabschnitts (144) verbunden ist, einen proximalen verjüngten Abschnitt (140), der mit einem proximalen Ende des Klappenhalteabschnitts (144) verbunden ist, einen distalen Schenkel (180), der mit einem distalen Ende des distalen verjüngten Abschnitts (142) verbunden ist, und einen proximalen Schenkel (182), der mit einem proximalen Ende des proximalen verjüngten Abschnitts (140) verbunden ist, aufweist;
Einführen einer distalen Schulter (160), die an einem ersten Schaft (104) der Zuführvorrichtung (100) montiert ist, durch den distalen Schenkel (180) und in den distalen verjüngten Abschnitt (142) des aufpumpbaren Ballons (108);
Einführen einer proximalen Schulter (120), die an einem zweiten Schaft (105) der Zuführvorrichtung (100) montiert ist, durch den proximalen Schenkel (182) und in den proximalen verjüngten Abschnitt (140) des aufpumpbaren Ballons (108);
Befestigen des distalen Schenkels (180) des aufpumpbaren Ballons (108) an der distalen Schulter (160); und
Befestigen des proximalen Schenkels (182) des aufpumpbaren Ballons (108) an dem zweiten Schaft (105) und/oder der proximalen Schulter (120),
wobei das Bilden des aufpumpbaren Ballons (108) umfasst:
Bilden des Klappenhalteabschnitts (144) in einer allgemein zylindrischen Form, deren Durchmesser kleiner ist als sowohl ein Durchmesser eines proximalen Endes des distalen verjüngten Abschnitts (142) als auch ein Durchmesser eines distalen Endes des proximalen verjüngten Abschnitts (140),
Bilden einer oder mehrerer axial verlaufender Falten (143, 145) entlang wenigstens von Abschnitten des distalen verjüngten Abschnitts (142) und des proximalen verjüngten Abschnitts (140),
Bilden einer radialen Falte (188) am distalen Ende des proximalen verjüngten Abschnitts (140), wobei die radiale Falte (188) einen stumpfen oder rechten Winkel in Bezug auf den Klappenhalteabschnitt (144) bildet, und
Bilden einer ersten radialen Falte (184), die mit einer zweiten radialen Falte (185) am proximalen Ende (150) des distalen verjüngten Abschnitts (142) verbunden ist, wobei die erste und die zweite radiale Falte (184, 185) eine Tasche bilden, die sich am proximalen Ende (150) des distalen verjüngten Abschnitts (142) nach distal erstreckt.

10. Verfahren nach Anspruch 9, ferner umfassend ein Anordnen eines Hemmelements (350) um den Klappenhalteabschnitt (144).

11. Verfahren nach einem der Ansprüche 9 bis 10, ferner umfassend ein Umschließen des aufpumpbaren Ballons (108) mit einer Abdeckung (200), wobei die Abdeckung (200) ein erstes Abdeckungsstück (202a) und ein zweites Abdeckungsstück (202b) umfasst, die komplementär strukturiert sind, so dass sie passend zusammengefügt werden können, um den aufpumpbaren Ballon (108) zu umschließen, und wobei die Abdeckung (200) eine innere Aussparung definiert, deren Geometrie im Wesentlichen zu einer Form des aufpumpbaren Ballons (108) im entleerten Zustand passt.

12. Verfahren nach einem der Ansprüche 9 bis 11, wobei ein proximales Ende der distalen Schulter (160) einen größeren Durchmesser aufweist als ein Durchmesser des distalen Schenkels (180), und das Einführen der distalen Schulter (160) durch den distalen Schenkel (180) ein radiales Zusammendrücken des proximalen Endes der distalen Schulter (160) umfasst.

13. Verfahren nach einem der Ansprüche 9 bis 12, wobei ein distales Ende der proximalen Schulter (170) einen größeren Durchmesser aufweist als ein Durchmesser des proximalen Schenkels (182), und das Einführen der proximalen Schulter (170) durch den proximalen Schenkel (182) das radiale Zusammendrücken des distalen Endes der proximalen Schulter (170) umfasst.

14. Klappenprothesen-Zuführanordnung, umfassend:
eine Zuführvorrichtung (100), umfassend:
einen Schaft (105); und
einen aufpumpbaren Ballon (108) nach einem der Ansprüche 1 bis 8, der in einem entleerten Zustand auf dem Schaft (105) montiert ist.

15. Klappenprothesen-Zuführanordnung nach Anspruch 14, die ferner ein Hemmelement (350) umfasst, das sich um eine Außenfläche des Klappenhalteabschnitts (144) erstreckt.

## Revendications

1. Ensemble ballonnet destiné à un dispositif de pose transcathéter conçu pour implanter un dispositif médical, comprenant :
un ballonnet gonflable (108) présentant un état gonflé et un état dégonflé, le ballonnet gonflable (108) dans l'état dégonflé comprenant :
une partie de retenue (144) de valvule présentant une forme généralement cylindrique et un ou plusieurs plis axiaux (146) ;
une partie effilée distale (142) reliée à une extrémité distale de la partie de retenue (144) de valvule, une extrémité proximale (150) de la partie effilée distale (142) présentant un diamètre (D1) plus grand que celui d'une extrémité distale (152) de la partie effilée distale (142) et de la partie de retenue (144) de valvule ;
une partie effilée proximale (140) reliée à une extrémité proximale de la partie de retenue de valvule, une extrémité distale (154) de la partie effilée proximale (140) présentant un diamètre (D2) plus grand que celui d'une extrémité proximale (156) de la partie effilée proximale (140) et de la partie de retenue de valvule (130) ;
une patte distale (180) reliée à l'extrémité distale (152) de la partie effilée distale (142) ; et
une patte proximale (182) reliée à l'extrémité proximale (156) de la partie effilée proximale (140) ;
la partie effilée distale (142) comprenant un ou plusieurs plis axiaux (143), la partie effilée proximale (140) comprenant un ou plusieurs plis axiaux (145),
l'extrémité proximale (150) de la partie effilée distale (142) comprenant un premier pli radial (184) relié à un second pli radial (185), les premier et second plis radiaux (184, 185) formant une poche s'étendant de manière distale au niveau de l'extrémité proximale (150) de la partie effilée distale (142) et
l'extrémité distale (154) de la partie effilée proximale (140) comprenant un pli radial (188) qui forme un angle obtus ou droit par rapport à la partie de retenue (144) de valvule.

2. Ensemble ballonnet selon la revendication 1, comprenant en outre un élément de restriction (350) s'étendant autour d'une surface externe de la partie de retenue (144) de valvule de façon à empêcher le dépliage dudit un ou desdits plusieurs plis axiaux (146) de la partie de retenue (144) de valvule.

3. Ensemble ballonnet selon l'une quelconque des revendications 1 à 2, comprenant en outre un recouvrement (200) qui renferme le ballonnet gonflable (108), le recouvrement (200) comprenant une partie d'extrémité proximale (210), une partie d'extrémité distale (206) et une partie intermédiaire (208) entre la partie d'extrémité proximale (210) et la partie d'extrémité distale (206), la partie d'extrémité proximale (210) définissant un évidement proximal qui est formé afin de correspondre sensiblement à une forme de la partie effilée proximale (140) du ballonnet gonflable (108), la partie d'extrémité distale (206) définissant un évidement distal qui est formé afin de correspondre sensiblement à une forme de la partie effilée distale (142) du ballonnet gonflable (108) et la partie intermédiaire (208) définissant un évidement intermédiaire qui est formé afin de correspondre sensiblement à une forme de la partie de retenue (144) de valvule du ballonnet gonflable (108).

4. Ensemble ballonnet selon la revendication 3, le recouvrement (200) comprenant une première pièce (202a) de recouvrement et une seconde pièce (202b) de recouvrement qui sont structurées de manière complémentaire de telle sorte qu'elles peuvent être assemblées ensemble par correspondance afin d'enfermer le ballonnet gonflable (108) dans un l'état dégonflé.

5. Ensemble ballonnet selon la revendication 4, comprenant en outre un manchon externe (204) s'étendant sur le recouvrement (200) de façon à empêcher la séparation des première et seconde pièces (202a, 202b) de recouvrement l'une de l'autre.

6. Ensemble ballonnet selon l'une quelconque des revendications 1 à 5, le ballonnet gonflable (108) comprenant une lumière interne s'étendant à travers la partie effilée proximale (140), la partie de retenue (144) de valvule et la partie effilée distale (142).

7. Ensemble ballonnet selon l'une quelconque des revendications 1 à 6, la partie effilée proximale (140) du ballonnet gonflable (108) comprenant une partie d'extrémité proximale,
ledit un ou lesdits plusieurs plis axiaux (145) de la partie effilée proximale (140) ne s'étendant pas dans la partie d'extrémité proximale de la partie effilée proximale (140).

8. Ensemble ballonnet selon l'une quelconque des revendications 1 à 7, la partie effilée distale (142) du ballonnet gonflable (108) comprenant une partie d'extrémité distale, ledit un ou lesdits plusieurs plis axiaux (143) de la partie effilée distale (142) ne s'étendant pas dans la partie d'extrémité distale de la partie effilée distale (142).

9. Procédé de formation d'un ballonnet (108) destiné à un dispositif de pose transcathéter conçu pour implanter un dispositif médical, le procédé comprenant :
la formation d'un ballonnet gonflable (108) dans un état dégonflé, le ballonnet (108) dans l'état dégonflé présentant une partie de retenue (144) de valvule, une partie effilée distale (142) reliée à une extrémité distale de la partie de retenue (144) de valvule, une partie effilée proximale (140) reliée à une extrémité proximale de la partie de retenue (144) de valvule, une patte distale (180) reliée à une extrémité distale de la partie effilée distale (142) et une patte proximale (182) reliée à une extrémité proximale de la partie effilée proximale (140) ;
l'insertion d'un épaulement distal (160) monté sur une première tige (104) du dispositif de pose (100) à travers la patte distale (180) et dans la partie effilée distale (142) du ballonnet gonflable (108) ;
l'insertion d'un épaulement proximal (120) monté sur une seconde tige (105) du dispositif de pose (100) à travers la patte proximale (182) et dans la partie effilée proximale (140) du ballonnet gonflable (108) ;
la fixation de la patte distale (180) du ballonnet gonflable (108) à l'épaulement distal (160) ; et
la fixation de la patte proximale (182) du ballonnet gonflable (108) à la seconde tige (105) et/ou à l'épaulement proximal (120),
la formation du ballonnet gonflable (108) comprenant :
la formation de la partie de retenue (144) de valvule dans une forme généralement cylindrique, dont le diamètre est inférieur à la fois à un diamètre d'une extrémité proximale de la partie effilée distale (142) et à un diamètre d'une extrémité distale de la partie effilée proximale (140),
la formation d'un ou de plusieurs plis (143, 145) s'étendant axialement le long d'au moins des sections de la partie effilée distale (142) et de la partie effilée proximale (140),
la formation d'un pli radial (188) au niveau de l'extrémité distale de la partie effilée proximale (140), le pli radial (188) formant un angle obtus ou droit par rapport à la partie de retenue (144) de valvule,
et
la formation d'un premier pli radial (184) relié à un second pli radial (185) au niveau de l'extrémité proximale (150) de la partie effilée distale (142), les premier et second plis radiaux (184, 185) formant une poche s'étendant de manière distale au niveau de l'extrémité proximale (150) de la partie effilée distale (142).

10. Procédé selon la revendication 9, comprenant en outre le placement d'un élément de restriction (350) autour de la partie de retenue (144) de valvule.

11. Procédé selon l'une quelconque des revendications 9 à 10, comprenant en outre l'enfermement du ballonnet gonflable (108) par un recouvrement (200), le recouvrement (200) comprenant une première pièce (202a) de recouvrement et une seconde pièce (202b) de recouvrement qui sont structurées de manière complémentaire de telle sorte qu'elles peuvent être assemblées ensemble par correspondance afin d'enfermer le ballonnet gonflable (108) et le recouvrement (200) définissant un évidement interne dont la géométrie correspond sensiblement à une forme du ballonnet gonflable (108) dans l'état dégonflé.

12. Procédé selon l'une quelconque des revendications 9 à 11, une extrémité proximale de l'épaulement distal (160) présentant un diamètre plus grand qu'un diamètre de la patte distale (180) et l'insertion de l'épaulement distal (160) à travers la patte distale (180) comprenant la compression radiale de l'extrémité proximale de l'épaulement distal (160).

13. Procédé selon l'une quelconque des revendications 9 à 12, une extrémité distale de l'épaulement proximal (170) présentant un diamètre plus grand qu'un diamètre de la patte proximale (182) et l'insertion de l'épaulement proximal (170) à travers la patte proximale (182) comprenant la compression radiale de l'extrémité distale de l'épaulement proximal (170).

14. Ensemble de pose de valvule prothétique, comprenant :
un dispositif de pose (100) comprenant :
une tige (105) ; et
un ballonnet gonflable (108) selon l'une quelconque des revendications 1 à 8 monté sur la tige (105) dans un état dégonflé.

15. Ensemble de pose de valvule prothétique selon la revendication 14, comprenant en outre un élément de restriction (350) s'étendant autour d'une surface externe de la partie de retenue (144) de valvule.
